# EUROPEAN PATENT SPECIFICATION

(11) **EP 1 351 954 B1**
(45) Date of publication and mention of the grant of the patent: **03.05.2006**
(21) Application number: 01995712.5
(22) Date of filing: 21.12.2001
(51) Int. Cl.: C07D 401/06, C07D 409/06, C07D 417/06, C07D 413/06, A61K 31/47, A61P 35/00

(54) **FARNESYL TRANSFERASE INHIBITING 4-HETEROCYCLYL-QUINOLINE AND QUINAZOLINE DERIVATIVES**
FARNESYLTRANSFERASEHEMMENDE 4-HETEROCYCLYLCHINOLIN- UND CHINAZOLINDERIVATE
DERIVES DE 4-HETEROCYCLYL-QUINOLINE ET QUINAZOLINE INHIBANT LAFARNESYL-TRANSFERASE

(30) Priority: 27.12.2000 EP 00204716
(43) Date of publication of application: 15.10.2003
(73) Proprietor: JANSSEN PHARMACEUTICA N.V., 2340 Beerse (BE)
(72) Inventor: ANGIBAUD, Patrick, René, c/o Janssen-Cilag S.A., F-92787 Issy-les-Moulineaux Cedex 9 (FR); VENET, Marc, Gaston, c/o Janssen-Cilag S.A., F-92787 Issy-les-Moulineaux Cedex 9 (FR); PONCELET, Virginie, S., c/o Janssen-Cilag S.A., F-92787 Issy-les-Moulineaux Cedex 9 (FR)
(74) Representative: Daelemans, Frank F.R.
(86) International application number: PCT/EP2001/015232
(87) International publication number: WO 2002/051834

(56) References cited:
- WO-A-00/47574
- US-A- 5 968 952
- US-A- 6 037 350
- US-A- 6 143 766

## Description

The present invention is concerned with novel 4- heterocyclyl quinoline and quinazoline derivatives, the preparation thereof, pharmaceutical compositions comprising said novel compounds and the use of these compounds as a medicine as well as methods of treatment by administering said compounds.

Oncogenes frequently encode protein components of signal transduction pathways which lead to stimulation of cell growth and mitogenesis. Oncogene expression in cultured cells leads to cellular transformation, characterized by the ability of cells to grow in soft agar and the growth of cells as dense foci lacking the contact inhibition exhibited by non-transformed cells. Mutation and/or overexpression of certain oncogenes is frequently associated with human cancer. A particular group of oncogenes is known as *ras* which have been identified in mammals, birds, insects, molluscs, plants, fungi and yeasts. The family of mammalian *ras* oncogenes consists of three major members ("isoforms") : H-*ras,* K-*ras* and N*-ras* oncogenes. These *ras* oncogenes code for highly related proteins generically known as p21^{*ras*}. Once attached to plasma membranes, the mutant or oncogenic forms of p21^{*ras*} will provide a signal for the transformation and uncontrolled growth of malignant tumor cells. To acquire this transforming potential, the precursor of the p21^{*ras*} oncoprotein must undergo an enzymatically catalyzed farnesylation of the cysteine residue located in a carboxyl-terminal tetrapeptide. Therefore, inhibitors of the enzymes that catalyzes this modification, i.e. farnesyl transferase, will prevent the membrane attachment of p21^{*ras*} and block the aberrant growth of *ras*-transformed tumors. Hence, it is generally accepted in the art that farnesyl transferase inhibitors can be very useful as anticancer agents for tumors in which *ras* contributes to transformation.

Since mutated oncogenic forms of *ras* are frequently found in many human cancers, most notably in more than 50 % of colon and pancreatic carcinomas (Kohl et al., *Science,* vol 260, 1834 - 1837, 1993), it has been suggested that farnesyl tranferase inhibitors can be very useful against these types of cancer.

In EP-0,371,564 there are described (1*H*-azol-1-ylmethyl) substituted quinoline and quinolinone derivatives which suppress the plasma elimination of retinoic acids. Some of these compounds also have the ability to inhibit the formation of androgens from progestines and/or inhibit the action of the aromatase enzyme complex.

In WO 97/16443, WO 97/21701, WO 98/40383 and WO 98/49157, there are described 2-quinolone derivatives which exhibit farnesyl transferase inhibiting activity. WO 00/39082 describes a class of novel 1,2-annelated quinoline compounds, bearing a nitrogen- or carbon-linked imidazole, which show farnesyl protein transferase and geranylgeranyl transferase inhibiting activity. Other quinolone compounds having farnesyl transferase inhibiting activity are described in WO 00/12498, 00/12499, 00/47574 and 01/53289.

Unexpectedly, it has been found that the present novel 4-heterocyclyl quinoline and quinazoline compounds show farnesyl protein transferase inhibiting activity.

The present invention concerns compounds of formula (I):- 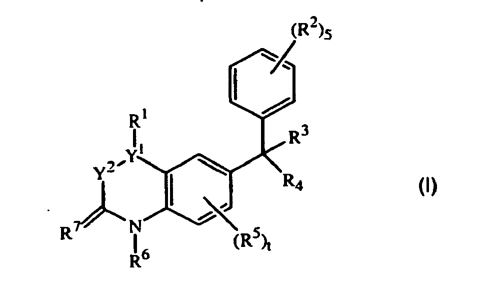 or a pharmaceutically acceptable salt or N-oxide or stereochemically isomeric form thereof, wherein
s is 0 or 1;
t is 0;
>Y¹-Y² - is a trivalent radical of formula

>C=N- (y-1)

or

>C=CR⁹- (y-2)

wherein R⁹ is hydrogen, halo, C₁₋₆alkyl, hydroxycarbonyl or C₁₋₆alkyloxycarbonyl;
- R¹: is a group of formula -Z-Het²
in which Z is a bond or a -(CH₂)₂- group and Het² is a monocyclic 5- or 6-membered heterocyclic ring containing one, two or three heteroatoms selected from oxygen, sulphur and nitrogen or a bicyclic 9- or 10-membered heterocyclic ring in which a benzene ring is fused to a heterocyclic ring containing one, two or three heteroatoms selected from oxygen, sulphur and nitrogen and optionally substituted by one or two substituents each independently selected from halo, C₁₋₆alkyl or phenyl;
- R²: is halo, cyano, nitro, -CHO, -CR²⁴=N-OR²⁵ in which R²⁴ is hydrogen and R²⁵ is hydrogen or C₁₋₆alkyl, or two R² substituents ortho to one another on the phenyl ring may independently form together a bivalent radical of formula (a-1);

-O-CH₂-O- (a-1)
- R³: is hydrogen or a group of formula (b-1) or (b-3)

-O-R¹⁰ (b-1)

-NR¹¹R¹² (b-3)

whereinR¹⁰ is hydrogen or a group of formula -Alk-OR¹³.
R¹¹ is hydrogen;
R¹² is hydrogen, C₁₋₆alkyl, C₁₋₆alkylcarbonyl, hydroxy or C₁₋₆alkyloxy;
Alk is C₁₋₆alkanediyl and R¹³ is hydrogen;
- R⁴: is a group of formula (c-2) or (c-3) of formula 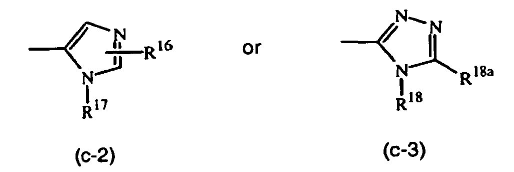 wherein R¹⁶ is hydrogen, halo or C₁₋₆alkyl,
R¹⁷ is hydrogen or C₁₋₆alkyl;
R¹⁸ is hydrogen or C₁₋₆alkyl;
R^{18a} is hydrogen;
- R⁶: is hydrogen, -(CR²⁰R²¹)ₚ-C₃₋₁₀cycloalkyl, -C₁₋₆alkylCO₂R²⁴ ;
-C₁₋₆alkyl CONR²² R²³, -Alk-Ar² or -AlkHet²or C₁₋₆alkyl; p is 0 to 5;
R²⁰ and R²¹ are independently hydrogen or C₁₋₆ alkyl and are independently defined for each iteration of p in excess of 1;
R²² and R²³ are independently hydrogen, C₁₋₆ alkyl or -(CR²⁰R²¹)ₚ-C₃₋₁₀cycloalkyl, or together with the adjacent nitrogen atom form a 5- or 6-membered heterocyclic ring optionally containing one, two or three further heteroatoms selected from oxygen, nitrogen or sulphur and optionally substituted by one or two substituents each independently selected from halo, hydroxy, cyano, nitro, C₁₋₆alkyl, haloC₁₋₆alkyl, C₁₋₆alkyloxy, OCF₃, hydroxycarbonyl, C₁₋₆alkyloxycarbonyl, aminocarbonyl, mono- or di-(C₁₋₆alkyl)aminocarbonyl, amino, mono- or di-(C₁₋₆alkyl)amino, C₁₋₆alkylsulfonylamino, oxime, or phenyl; R²⁴ is hydrogen, C₁₋₆ alkyl, -(CR₂₀R₂₁)ₚ-C₃₋₁₀cycloalkyl or arylC₁₋₆alkyl;
- R⁷: is oxygen or sulphur; or R⁶ and R⁷ together form a trivalent radical of formula (x-1), (x-2), (x-3), (x-4) or (x-9):

-CR³⁰=CR³¹-N= (x-1)

-CR³⁰=N-N= (x-2)

-C(=O)-NH-N= (x-3)

-N=N-N= (x-4)

or

-N=N-CR³⁰= (x-9)

wherein each R³⁰, R³¹ and R³² are independently hydrogen, C₁₋₆ alkyl, -OR²⁴, -COOR²⁴, -NR²²R²³, -C₁₋₆ alkylOR²⁴, -C₁₋₆ alkylSR²⁴, R²³R²²NC₁₋₆alkyl-, -CONR²²R²³, C₂₋₆alkenyl, C₂₋₆alkenylAr², C₂₋₆alkenylHet², cyano, amino, thio, C₁₋₆ alkylthio, -O-Ar², -S-Ar² or Ar²;
- Ar²: is phenyl, naphthyl or phenyl or naphthyl substituted by one to five substituents each independently selected from halo, hydroxy, cyano, nitro, C₁₋₆alkyl, haloC₁₋₆alkyl. - C₁₋₆alkylNR²²R²³, C₁₋₆alkyloxy, OCF₃, hydroxycarbonyl, C₁₋₆alkyloxycarbonyl, aryloxy, -NR²²R²¹, C₁₋₆alkylsulfonylamino, oxime or phenyl, or a bivalent substituent of formula -O-CH₂-O- or -O-CH₂-CH₂-O-.

As used in the foregoing definitions and hereinafter, halo is generic to fluoro, chloro, bromo and iodo; C₁₋₄alkyl defines straight and branched chain saturated hydrocarbon radicals having from 1 to 4 carbon atoms such as, e.g. methyl, ethyl, propyl, butyl, 1-methylethyl, 2-methylpropyl and the like; C₁₋₆alkyl includes C₁₋₄alkyl and the higher homologues thereof having 5 to 6 carbon atoms such as, for example, pentyl, 2-methylbutyl, hexyl, 2-methylpentyl and the like; C₁₋₆alkanediyl defines bivalent straight and branched chained saturated hydrocarbon radicals having from 1 to 6 carbon atoms, such as, for example, methylene, 1,2-ethanediyl, 1,3-propanediyl, 1,4-butanediyl, 1,5-pentanediyl, 1,6-hexanediyl and the branched isomers thereof; haloC₁₋₆alkyl defines C₁₋₆alkyl containing one or more halo substituents for example trifluoromethyl; C₂₋₆alkenyl defines straight and branched chain hydrocarbon radicals containing one double bond and having from 2 to 6 carbon atoms such as, for example, ethenyl, 2-propenyl, 3-butenyl, 2-pentenyl, 3-pentenyl, 3-methyl-2-butenyl, and the like. The term "S(O)" refers to a sulfoxide and "S(O)₂" to a sulfone. Aryl defines phenyl, naphthalenyl or phenyl substituted with one or more substituents each independently selected from halo, C₁₋₆alkyl, C₁₋₆alkyloxy or trifluoromethyl, cyano, hydroxycarbonyl. The term "bicyclic heterocyclic ring" used in relation to the definition of Het² includes bicyclic ring systems in which a heterocyclic ring is fused to a benzene ring providing the bicyclic ring system is bonded to the remainder of the molecule via the heterocyclic ring.

The pharmaceutically acceptable acid addition salts as mentioned hereinabove are meant to comprise the therapeutically active non-toxic acid addition salt forms which the compounds of formula (I) are able to form. The compounds of formula (I) which have basic properties can be converted in their pharmaceutically acceptable acid addition salts by treating said base form with an appropriate acid. Appropriate acids comprise, for example, inorganic acids such as hydrohalic acids, e.g. hydrochloric or hydrobromic acid; sulfuric; nitric; phosphoric and the like acids; or organic acids such as, for example, acetic, propanoic, hydroxyacetic, lactic, pyruvic, oxalic, malonic, succinic (*i.e.* butane-dioic acid), maleic, fumaric, malic, tartaric, citric, methanesulfonic, ethanesulfonic, benzenesulfonic, *p*-toluenesulfonic, cyclamic, salicylic, *p*-amino-salicylic, pamoic and the like acids.

The term acid addition salts also comprises the hydrates and the solvent addition forms which the compounds of formula (I) are able to form. Examples of such forms are e.g. hydrates, alcoholates and the like.

The term stereochemically isomeric forms of compounds of formula (I), as used hereinbefore, defines all possible compounds made up of the same atoms bonded by the same sequence of bonds but having different three-dimensional structures which are not interchangeable, which the compounds of formula (I) may possess. Unless otherwise mentioned or indicated, the chemical designation of a compound encompasses the mixture of all possible stereochemically isomeric forms which said compound may possess. Said mixture may contain all diastereomers and/or enantiomers of the basic molecular structure of said compound. All stereochemically isomeric forms of the compounds of formula (I) both in pure form or in admixture with each other are intended to be embraced within the scope of the present invention.

Some of the compounds of formula (I) may also exist in their tautomeric forms. Such forms although not explicitly indicated in the above formula are intended to be included within the scope of the present invention.

Whenever used hereinafter, the term "compounds of formula (I)" is meant to include also the pharmaceutically acceptable acid addition salts and all stereoisomeric forms.

A particular group of compounds consists of those compounds of formula (I) wherein s is 1, t is 0, >Y¹-Y² is a trivalent radical of formula (y-1) or (y-2), R¹ is a group of formula -Z-Het² in which Z is a bond or a -(CH₂)₂- group and Het² is a monocyclic 5- or 6-membered heterocyclic ring containing one, two or three heteroatoms selected from oxygen, sulphur and nitrogen or a bicyclic 9- or 10-membered heterocyclic ring in which a benzene ring is fused to a heterocyclic ring containing one, two or three heteroatoms selected from oxygen, sulphur and nitrogen and optionally substituted by one or two substituents each independently selected from halo, C₁₋₆alkyl or phenyl,
R² is halo or cyano, R³ is hydrogen or a radical of formula (b-1) or (b-3) wherein R¹⁰ is hydrogen or -Alk-OR¹³, R¹¹ is hydrogen, R¹² is hydrogen or C₁₋₆alkylcarbonyl and R¹³ is hydrogen; R⁴ is a radical of formula (c-2) or (c-3) wherein R¹⁶ is hydrogen, R¹⁷ is C₁₋₆alkyl, R¹⁸ is C₁₋₆alkyl and R^{18a} is hydrogen;
R⁶ is hydrogen, C₁₋₆alkyl,
-CH₂ -C₃₋₁₀cycloalkyl,
-C₁₋₆alkylCO₂R²⁴ (R²⁴=H or Et), aminocarbonylC₁₋₆alkyl, -Alk-Ar² or -AlkHet² ;
R⁷ is oxygen or sulphur; or R⁶ and R⁷ together form a trivalent radical of formula (x-2), (x-3) or (x-4).

More preferred compounds are those compounds of formula (1) wherein s is 1, t is 0, >Y¹-Y² is a trivalent radical of formula (y-1) or (y-2), R¹ is a group of formula -Z-Het² in which Z is a bond or a -(CH₂)₂- group and Het² is a monocyclic 5- or 6-membered heterocyclic ring containing one, two or three heteroatoms selected from oxygen, sulphur and nitrogen especially selected from imidazolyl, thiophene, pyridyl, oxazolyl, thiazolyl and oxadiazolyl, R² is halo, preferably 4-chloro or 4-fluoro, or cyano, preferably 4-cyano, R³ is hydrogen or a radical of formula (b-1) or (b-3) wherein R⁹ is hydrogen, R¹⁰ is hydrogen, R¹¹ is hydrogen and R¹² is hydrogen or C₁₋₆alkylcarbonyl; R⁴ is a radical of formula (c-2) or (c-3) wherein R¹⁶ is hydrogen, R¹⁷ is C₁₋₆alkyl, R¹⁸ is C₁₋₆alkyl and R^{18a} is hydrogen; R⁶ is hydrogen, C₁₋₆alkyl,
-CH₂-C₃₋₁₀cycloalkyl or -C₁₋₆alkylAr²;
R⁷ is oxygen or sulphur; or R⁶ and R⁷ together form a trivalent radical of formula (x-2) or (x-4).

Especially preferred compounds are those compounds of formula (I) wherein s is 1, t is 0, >Y¹-Y² is a trivalent radical of formula (y-1) or (y-2), R¹ is a group of formula -Z-Het² in which Z is a bond or a -(CH₂)₂- group and Het² is selected from thiophene particularly 2-thiophene, pyridyl particularly 3-pyridyl and thiazolyl particularly 1,3-thiazol-2-yl, optionally substituted by chloro, methyl or phenyl, R² is halo, preferably chloro, and most preferably 4-chloro, or cyano, preferably 4-cyano, R³ is a radical of formula (b-1) or (b-3) wherein R⁹ is hydrogen, R¹⁰ and R¹¹ are hydrogen and R¹² is hydrogen or C₁₋₆alkylcarbonyl; R⁴ is a radical of formula (c-2) or (c-3) wherein R¹⁶ is hydrogen, R¹⁷ is C₁₋₆alkyl preferably methyl, R¹⁸ is C₁₋₆alkyl preferably methyl and R^{18a} is hydrogen; R⁶ is hydrogen, C₁₋₆alkyl, -CH₂-C₃₋₁₀cycloalkyl or -C₁₋₆alkylAr²; R⁷ is oxygen or sulphur; or R⁶ and R⁷ together form a trivalent radical of formula (x-4).

The most preferred compounds according to the invention are:-
6-[(4-chlorophenyl)hydroxy(1-methyl-1*H*-imidazol-5-yl)methyl]-1-methyl-4-(2-thienyl)- 2(1*H*)-quinolinone
6-[(4-chlorophenyl)hydroxy(1-methyl-1*H*-imidazol-5-yl)methyl]4-(3-pyridinyl)-2(1*H*)-quinolinone,
6-[(4-chlorophenyl)hydroxy(1-methyl-1*H*-imidazol-5-yl)methyl]-1-methyl-4-(4-methyl-2-thiazolyl)-2(1*H*)-quinolinone
*N*-[(4-chlorophenyl)[1,2-dihydro-1-methyl-2-oxo-4-(4-phenyl-2-thiazolyl)-6-quinolinyl](1-methyl-1*H*-imidazol-5-yl)methyl]- acetamide and
6-[amino(4-chlorophenyl)(1-methyl-1*H*-imidazol-5-yl)methyl]-4-[2-(5-chloro-2-thienyl)ethyl]-1-methyl-2(1*H*)-quinolinone
and their pharmaceutically acceptable salts.

The compounds of formula (I) and their pharmaceutically acceptable salts and N-oxides and stereochemically isomeric forms thereof may be prepared in conventional manner, for example by a process which comprises:-

### a) cyclising a compound of formula (II):

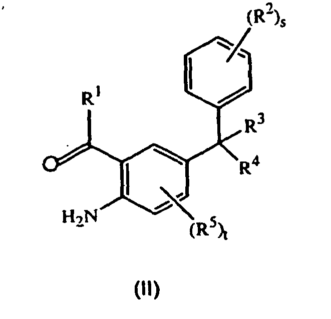

- R¹ is a group of formula -Z-Het² in which Z is a bond or a -(CH₂)₂- group and Het² is a monocyclic 5- or 6-membered heterocyclic ring containing one or more heteroatoms selected from oxygen, sulphur and nitrogen or a bicyclic 9- or 10-membered heterocyclic ring in which a benzene ring is fused to a heterocyclic ring containing one or more heteroatoms selected from oxygen, sulphur and nitrogen and optionally substituted by one or two substituents each independently selected from halo, hydroxy, cyano, nitro, C₁₋₆alkyl, haloC₁₋₆alkyl, - C₁₋₆alkylNR²²R²³, C₁₋₆alkyloxy, OCF₃, hydroxycarbonyl, C₁₋₆alkyloxycarbonyl, -CONR²²R²³, -NR²²R²³, C₁₋₆alkylsulfonylamino, oxime or phenyl.
- R² is halo, cyano, nitro, C₁₋₆alkyl, cyanoC₁₋₆alkyl, -C₁₋₆alkyl NR²²R²³; cyanoC₂₋₆alkenyl, -NR²²R²³, -CHO, -CR²⁴=N-OR²⁵, C₁₋₆alkyloxycarbonyl, -CONR²²R²³; or
   two R² substituents adjacent to one another on the phenyl ring may independently form together a bivalent radical of formula

   -O-CH₂-O- (a-1)

   -O-CH₂-CH₂-O- (a-2)
- R³ is hydrogen, C₁₋₆alkyl, -(CR²⁰R²¹)ₚ-C₃₋₁₀cycloalkyl, haloC₁₋₆alkyl, cyanoC₁₋₆alkyl, hydroxyC₁₋₆alkyl, C₁₋₆alkyloxyC₁₋₆alkyl, -C₁₋₆alkyl NR²²R²³, Het²C₁₋₆alkyl, -C₂₋₆alkenyl NR²²R²³ or -Het² ; or a group of formula

   -O-R¹⁰ (b-1)

   -NR¹¹R¹² (b-3)

   wherein R¹⁰ is hydrogen, C₁₋₆alkyl, or -(CR²⁰R²¹)ₚ-C₃₋₁₀cycloalkyl, or a group of formula -Alk-OR¹³ or -Alk-NR¹⁴R¹⁵;
   R¹¹ is hydrogen or C₁₋₆alkyl;
   R¹² is hydrogen, hydroxy, C₁₋₆alkyl, -(CR²⁰R²¹)ₚ-C₃₋₁₀cycloalkyl,
   C₁₋₆alkyloxy, C₁₋₆alkylcarbonyl, arylC₁₋₆alkylcarbonyl,
   Het²C₁₋₆alkylcarbonyl, aminocarbonyl, or a radical of formula
   -Alk-OR¹³ or Alk-NR¹⁴R¹⁵;
   wherein Alk is C₁₋₆alkanediyl;
   R¹³ is hydrogen, C₁₋₆alkyl or -(CR²⁰R²¹)ₚ-C₃₋₁₀cycloalkyl;
   R¹⁴ is hydrogen, C₁₋₆alkyl, or-(CR²⁰R²¹)ₚ-C₃₋₁₀cycloalkyl;
   R¹⁵ is hydrogen or C₁₋₆alkyl;
- R⁴ is a radical of formula (c-2) or (c-3)
   wherein R¹⁶ is hydrogen, halo or C₁₋₆alkyl,
   R¹⁷ is hydrogen, C₁₋₆alkyl, -(CR²⁰R²¹)p-C₃₋₁₀cycloalkyl,
   C₁₋₆alkyloxyC₁₋₆alkyl or trifluoromethyl;
   R¹⁸ is hydrogen, C₁₋₆alkyl or -(CR²⁰R²¹)ₚ-C₃₋₁₀cycloalkyl;
   R^{18a} is hydrogen;
- R⁵ is cyano, halo, C₁₋₆alkyl, C₂₋₆alkynyl, C₁₋₆alkyloxy or C₁₋₆alkyloxycarbonyl:
- R⁶ is hydrogen, C₁₋₆alkyl, -C₁₋₆alkylCO₂R²⁴, -C₁₋₆alkyl CONR²² R²³ , -Alk-Ar², -AlkHet²or -(CR²⁰R²¹)ₚ-C₃₋₁₀cycloalkyl,
- k⁷ is oxygen or sulphur; or R⁶ and R⁷ together form a trivalent radical of formula (x-1), (x-2), (x-3), (x-4) or (x-9)

A group of interesting compounds consists of those compounds of formula (I) wherein one or more of the following restrictions apply:
- s is 0 or 1;
- t is 0;
- >Y¹-Y²- is a trivalent radical of formula (y-1) or (y-2), wherein R⁹ is hydrogen, halo, C₁₋₄alkyl, hydroxycarbonyl, or C₁₋₄alkyloxycarbonyl;
- R¹ is a group of formula -Z-Het² in which Z is a bond or a -(CH₂)₂- group and Het² is a monocyclic 5- or 6-membered heterocyclic ring containing one, two or three heteroatoms selected from oxygen, sulphur and nitrogen or a bicyclic 9- or 10-membered heterocyclic ring in which a benzene ring is fused to a heterocyclic ring containing one, two or three heteroatoms selected from oxygen, sulphur and nitrogen and optionally substituted by one or two substituents each independently selected from halo, C₁₋₆alkyl or phenyl.
- R² is halo, cyano, nitro, -CHO, -CR²⁴=N-OR²⁵ in which R²⁴ is hydrogen and R²⁵ is hydrogen or C₁₋₆alkyl, or two R² substituents ortho to one another on the phenyl ring may independently form together a bivalent radical of formula (a-1);
- R³ is hydrogen or a group of formula (b-1) or (b-3) wherein
   R¹⁰ is hydrogen or a group of formula -Alk-OR¹³.
   R¹¹ is hydrogen;
   R¹² is hydrogen, C₁₋₆alkyl, C₁₋₆alkylcarbonyl, hydroxy or C₁₋₆alkyloxy;
   Alk is C₁₋₆alkanediyl and R¹³ is hydrogen;
- R⁴ is a group of formula (c-2) or (c-3) wherein
   R¹⁶ is hydrogen, halo or C₁₋₆alkyl;
   R¹⁷ is hydrogen or C₁₋₆alkyl;
   R¹⁸ is hydrogen or C₁₋₆alkyl;
   R^{18a} is hydrogen;
- R⁶ is hydrogen, -(CR²⁰R²¹)ₚ-C₃₋₁₀cycloalkyl, -C₁₋₆alkylCO₂R²⁴,
   -C₁₋₆alkyl CONR²² R²³, -Alk-Ar² or -AlkHet² or C₁₋₆alkyl;
- R⁷ is oxygen or sulphur; or R⁶ and R⁷ together form a trivalent radical of formula (x-1), (x-2), (x-3), (x-4) or (x-9);
- aryl is phenyl.

A particular group of compounds consists of those compounds of formula (I) wherein s is 1, t is 0, >Y¹-Y² is a trivalent radical of formula (y-1) or (y-2), R¹ is a group of formula -Z-Het² in which Z is a bond or a -(CH₂)₂- group and Het² is a monocyclic 5-or 6-membered heterocyclic ring containing one, two or three heteroatoms selected from oxygen, sulphur and nitrogen or a bicyclic 9- or 10-membered heterocyclic ring in which a benzene ring is fused to a heterocyclic ring containing one, two or three heteroatoms selected from oxygen, sulphur and nitrogen and optionally substituted by one or two substituents each independently selected from halo, C₁₋₆alkyl or phenyl, R² is halo or cyano, R³ is hydrogen or a radical of formula (b-1) or (b-3) wherein R¹⁰ is hydrogen or -Alk-OR¹³, R¹¹ is hydrogen, R¹² is hydrogen or C₁₋₆alkylcarbonyl and R¹³ is hydrogen; R⁴ is a radical of formula (c-2) or (c-3) wherein R¹⁶ is hydrogen, R¹⁷ is C₁₋₆alkyl, R¹⁸ is C₁₋₆alkyl and R^{18a} is hydrogen;
R⁶ is hydrogen, C₁₋₆alkyl,
-CH₂ -C₃₋₁₀cycloalkyl,
-C₁₋₆alkylCO₂R²⁴ (R²⁴=H or Et), aminocarbonylC₁₋₆alkyl, -Alk-Ar² or -AlkHet²;
R⁷ is oxygen or sulphur; or R⁶ and R⁷ together form a trivalent radical of formula (x-2), (x-3) or (x-4).

More preferred compounds are those compounds of formula (I) wherein s is 1, t is 0, >Y¹-Y² is a trivalent radical of formula (y-1) or (y-2), R¹ is a group of formula -Z-Het² in which Z is a bond or a -(CH₂)₂- group and Het² is a monocyclic 5- or 6-membered heterocyclic ring containing one, two or three heteroatoms selected from oxygen, sulphur and nitrogen especially selected from imidazolyl, thiophene, pyridyl, oxazolyl, thiazolyl and oxadiazolyl, R² is halo, preferably 4-chloro or 4-fluoro, or cyano, preferably 4-cyano, R³ is hydrogen or a radical of formula (b-1) or (b-3) wherein R⁹ is hydrogen, R¹⁰ is hydrogen, R¹¹ is hydrogen and R¹² is hydrogen or C₁₋₆alkylcarbonyl;
R⁴ is a radical of formula (c-2) or (c-3) wherein R¹⁶ is hydrogen, R¹⁷ is C₁₋₆alkyl, R¹⁸ is C₁₋₆alkyl and R^{18a} is hydrogen; R⁶ is hydrogen, C₁₋₆alkyl,
-CH₂-C₃₋₁₀cycloalkyl or -C₁₋₆alkylAr²;
R⁷ is oxygen or sulphur; or R⁶ and R⁷ together form a trivalent radical of formula (x-2) or (x-4).

Especially preferred compounds are those compounds of formula (I) wherein s is 1, t is 0, >Y¹-Y² is a trivalent radical of formula (y-1) or (y-2), R¹ is a group of formula -Z-Het² in which Z is a bond or a -(CH₂)₂- group and Het² is selected from thiophene particularly 2-thiophene, pyridyl particularly 3-pyridyl and thiazolyl particularly 1,3-thiazol-2-yl, optionally substituted by chloro, methyl or phenyl, R² is halo, preferably chloro, and most preferably 4-chloro, or cyano, preferably 4-cyano, R³ is a radical of formula (b-1) or (b-3) wherein R⁹ is hydrogen, R¹⁰ and R¹¹ are hydrogen and R¹² is hydrogen or C₁₋₆alkylcarbonyl; R⁴ is a radical of formula (c-2) or (c-3) wherein R¹⁶ is hydrogen, R¹⁷ is C₁₋₆alkyl preferably methyl, R¹⁸ is C₁₋₆alkyl preferably methyl and R^{18a} is hydrogen; R⁶ is hydrogen, C₁₋₆alkyl, -CH₂-C₃₋₁₀cycloalkyl or -C₁₋₆alkylAr²; R⁷ is oxygen or sulphur; or R⁶ and R⁷ together form a trivalent radical of formula (x-4).

The most preferred compounds according to the invention are:-
6-[(4-chlorophenyl)hydroxy(1-methyl-1*H*-imidazol-5-yl)methyl]-1-methyl-4-(2-thienyl)- 2(1*H*)-quinolinone
6-[(4-chlorophenyl)hydroxy(1-methyl-1*H*-imidazol-5-yl)methyl]-4-(3-pyridinyl)- 2(1*H*)-quinolinone,
6-[(4-chlorophenyl)hydroxy(1-methyl-1*H*-imidazol-5-yl)methyl]-1-methyl-4-(4-methyl-2-thiazolyl)- 2(1*H*)-quinolinone
*N*-[(4-chlorophenyl)[1,2-dihydro-1-methyl-2-oxo-4-(4-phenyl-2-thiazolyl)-6-quinolinyl](1-methyl-1*H*-imidazol-5-yl)methyl]- acetamide and
6-[amino(4-chlorophenyl)(1-methyl-1*H*-imidazol-5-yl)methyl]-4-[2-(5-chloro-2-thienyl)ethyl]-1-methyl-2(1*H*)-quinolinone
and their pharmaceutically acceptable salts.

The compounds of formula (I) and their pharmaceutically acceptable salts and N-oxides and stereochemically isomeric forms thereof may be prepared in conventional manner, for example by a process which comprises:- a) cyclising a compound of formula (II): 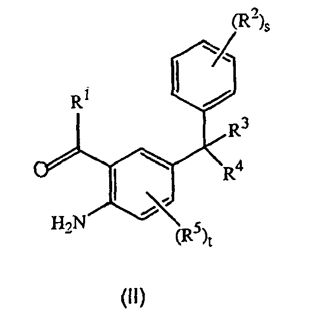 with a reagent serving to form a compound of formula (I) in which Z is a bond, R⁶ is hydrogen and R⁷ is oxygen;

### b) reacting a compound of formula (III):

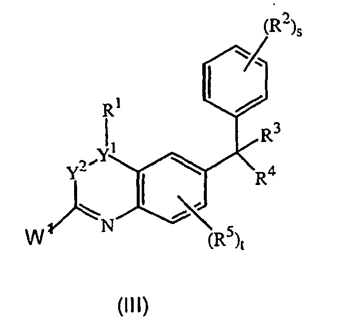

in which W¹ represents a replaceable or reactive group, with a reagent serving either to react with or replace the W¹ group in compound (III) to form a compound of formula (I) in which R⁶ is hydrogen and R⁷ is an oxygen or sulphur group or to react with the W¹ group and the adjacent nitrogen atom to form directly or indirectly a compound of formula (I) in which R⁶ and R⁷ together form a trivalent radical selected from formulae (x-1) to (x-10); or

### c) reacting a compound of formula (IV):

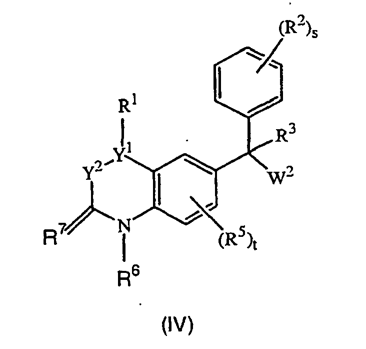

in which W² is a replaceable group, with an imidazole reagent serving to replace the group W² with an R⁴ group of formula (c-1); or

### d) reacting a compound of formula (V):

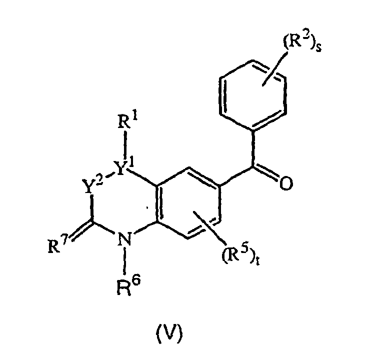

with an imidazole reagent to form a compound of formula (I) in which R⁴ is a group of formula (*c*-2)), or with a 3-mercapto-4-C₁₋₆alkyl-1,2,4-triazole reagent to form the corresponding 3-mercapto-4-C₁₋₆alkyl-1,2,4-triazole derivative, which is optionally methylated to form the corresponding 3-methylmercapto derivative, and subsequently removing the 3-mercapto or 3-methylmercapto group to form a compound of formula (I) in which R⁴ is a group of formula (c-3) in which R¹⁸ is a C₁₋₆alkyl group; or with a 3-bromopyridyl reagent to form a compound of formula (I) wherein R⁴ is a group of formula (c-4); or

### e) reacting a compound of formula (VI):

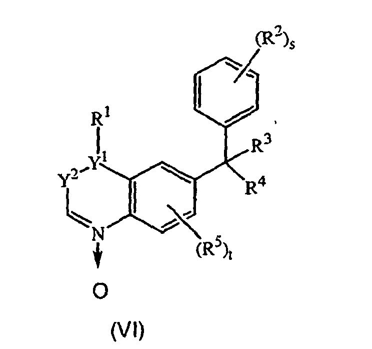

with a reagent serving to convert the said compound (VI) to a compound of formula (I) in which R⁶ is hydrogen and R⁷ is oxygen;
and optionally effecting one or more of the following conversions in any desired order:-
(i) converting a compound of formula (I) into a different compound of formula (I);
(ii) converting a compound of formula (I) in to a pharmaceutically acceptable salt or N-oxide thereof;
(iii) converting a pharmaceutically acceptable salt or N-oxide of a compound of formula (I) into the parent compound of formula (I);
(iv) preparing a stereochemical isomeric form of a compound of formula (I) or a pharmaceutically acceptable salt or N-oxide thereof.

With regard to process a), this can be effected in an analogous manner to that described for example in WO 97/21701 and WO98/49157 referred to above. Thus, the cyclisation may be effected for example by subjecting the compound of formula (II) to an acetylation reaction, e.g. by treatment with the anhydride of a carboxylic acid, e.g. acetic anhydride in a reaction-inert solvent, e.g. toluene, and subsequent reaction with a base such as potassium *tert*-butoxide in a reaction-inert solvent such as 1,2-dimethoxyethane.

With regard to process b), this can also be effected in an analogous manner to that described for example in WO 97/21701 and WO98/49157 referred to above for the preparation of compounds in which R⁷ is oxygen, for example by hydrolysis of an ether of formula (II) in which W¹ is C₁₋₆alkyloxy in an aqueous acid solution such hydrochloric acid.

With regard to process b), for the preparation of compounds in which R⁶ and R⁷ together form a trivalent radical of formula (x-1) to (x-10), this can be effected in an analogous manner to that described for example in WO 00/39082 referred to above. For example, when W¹ is chloro, the compound of formula (III) can be reacted with an azide compound for example sodium azide to form a corresponding compound of formula (I) in which R⁶ and R⁷ together form a trivalent radical of formula (x-4.).

With regard to process c), this can be effected for example by N-alkylating an intermediate of formula (IV), wherein W² is an appropriate leaving group such as, for example, chloro, bromo, methanesulfonyloxy or benzenesulfonyloxy, with an intermediate of formula (IVa) to form a compound of formula (I) in which R⁴ is a group of formula (c-1) represented by compounds of formula (I-a):

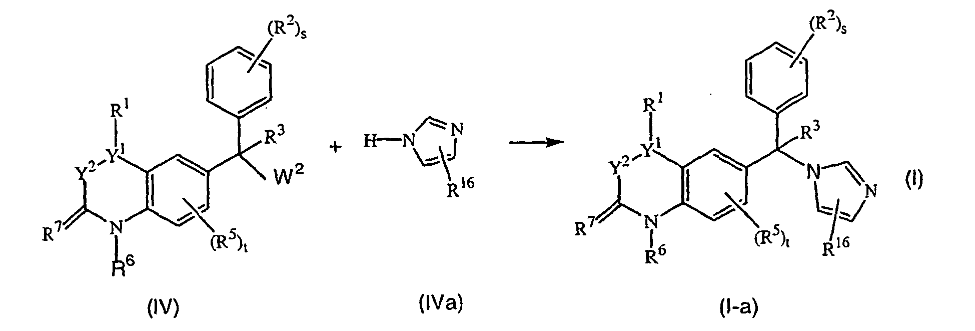

The reaction can be performed in a reaction-inert solvent such as, for example, acetonitrile, and optionally in the presence of a suitable base such as, for example, sodium carbonate, potassium carbonate or triethylamine. Stirring may enhance the rate of the reaction. The reaction may conveniently be carried out at a temperature ranging between room temperature and reflux temperature.

Also, compounds of formula (I-a) can be prepared by reacting an intermediate of formula (V) in which W² is hydroxy with an intermediate of formula (X), wherein Y is oxygen or sulfur, such as, for example, a 1,1'-carbonyldiimidazole.

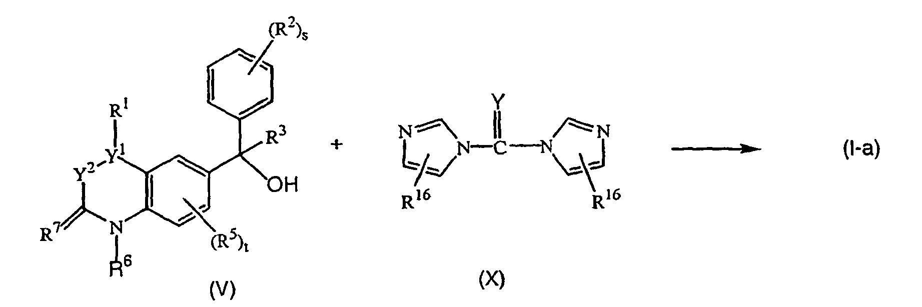

Said reaction may conveniently be conducted in a reaction-inert solvent, such as, e.g. tetrahydrofuran, optionally in the presence of a base, such as sodium hydride, and at a temperature ranging between room temperature and the reflux temperature of the reaction mixture.

With regard to process d), the compounds of formula (I) wherein R⁴ represents a radical of formula (c-2), R is hydroxy and R¹⁷ is C₁₋₆alkyl, said compounds being referred to as compounds of formula (I-b-1) may be prepared by reacting an intermediate ketone of formula (V) with an intermediate of formula (III-1). Said reaction requires the presence of a suitable strong base, such as, for example, butyl lithium in an appropriate solvent, such as, for example, tetrahydrofuran, and the presence of an appropriate silane derivative, such as, for example, triethylchlorosilane. During the work-up procedure an intermediate silane derivative is hydrolysed. Other procedures with protective groups analogous to silane derivatives can also be applied.

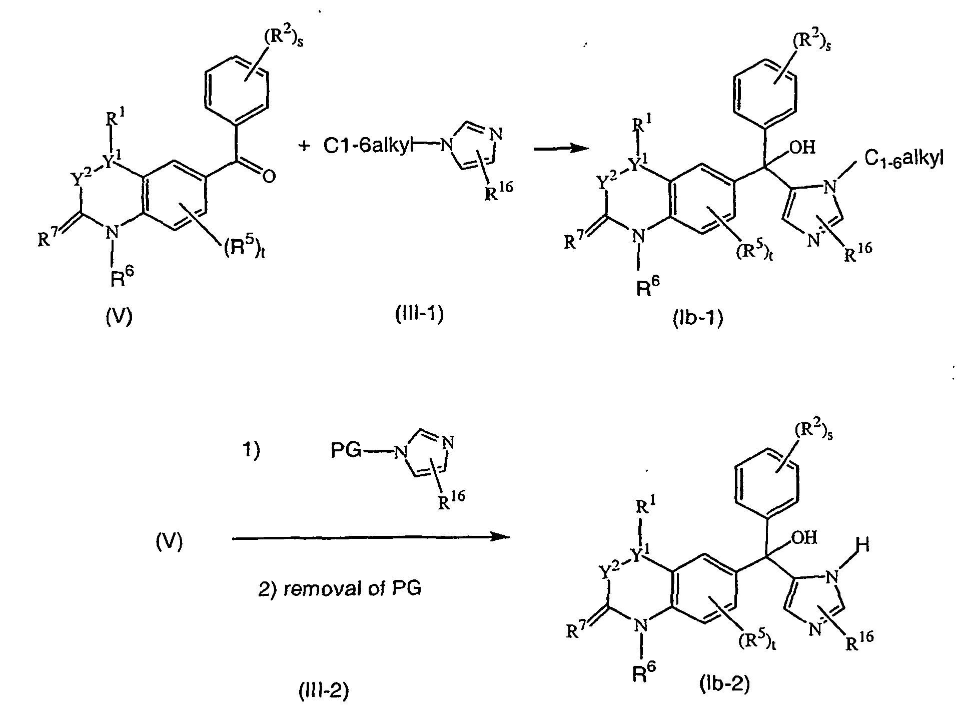

Also, the compounds of formula (I), wherein R⁴ is a radical of formula (c-2), R³ is hydroxy and R¹⁷ is hydrogen, said compounds being referred to as compounds of formula (I-b-2) may be prepared by reacting an intermediate ketone of formula (V) with a intermediate of formula (III-2), wherein PG is a protective group such as, for example, a sulfonyl group, e.g. a dimethylamino sulfonyl group, which can be removed after the addition reaction. Said reaction is conducted analogously as for the preparation of compounds of formula (I-b-1), followed by removal of the protecting group PG, yielding compounds of formula (I-b-2).

Also with regard to process d), the compounds of formula (I) wherein R⁴ represents a radical of formula (c-3) may be prepared by reacting the compound of formula (IV) with the triazole reagent, preferably in a reaction-inert solvent such as tetrahydrofuran, in the presence of a strong base such as butyl lithium at a temperature ranging from -78°C to room temperature. When the 3-mercapto derivative is methylated, this is conveniently effected with methyl iodide in the presence of a base such as sodium methylate. Removal of the 3-mercapto group is conveniently effected with sodium nitrite, for example in THF/H₂O in the presence of nitric acid. Removal of the 3-methylmercapto group is conveniently effected with Raney Nickel in ethanol or acetone. The compounds of formula (I) wherein R⁴ represents a radical of formula (c-4) may be prepared by reacting the compound of formula (IV) with the 3-bromopyridyl reagent , preferably in a reaction-inert solvent such as tetrahydrofuran, in the presence of a strong base such as butyl lithium at a temperature ranging from -78°C to room temperature.

With regard to process e), this may be effected for example as described in WO 97/21701 referred to above, by reacting the nitrone of formula (VI) with the anhydride of a carboxylic acid, e.g. acetic anhydride, thus forming the corresponding ester on the 2-position of the quinoline moiety, which ester can then be hydrolysed *in situ* to the corresponding quinolinone using a base such potassium carbonate. Alternatively the above nitrone can be reacted with tosyl chloride to prepare the corresponding tosylate which can then be hydrolysed *in situ.*

Examples of the interconversion of one compound of formula (I) into a different compound of formula (I) include the following reactions:-
a) compounds of formula (I-b) can be converted to compounds of formula (I-c), defined as a compound of formula (I) wherein R⁴ is a radical of formula (c-2) and R³ is hydrogen, by submitting the compounds of formula (I-b) to appropriate reducing conditions, such as, e.g. stirring in acetic acid in the presence of formamide, or treatment with sodium borohydride/ trifluoroacetic acid. 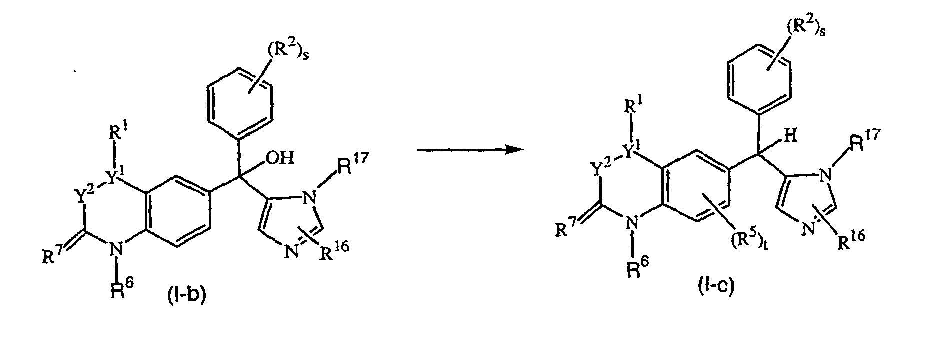
b) compounds of formula (I-b) can be converted to compounds of formula (I-f) wherein R³ is halo, by reacting the compounds of formula (I-b) with a suitable halogenating agent, such as, e.g. thionyl chloride or phosphorus tribromide. Successively, the compounds of formula (I-f) can be treated with a reagent of formula H-NR¹¹R¹² in a reaction-inert solvent, thereby yielding compounds of formula (I-g). 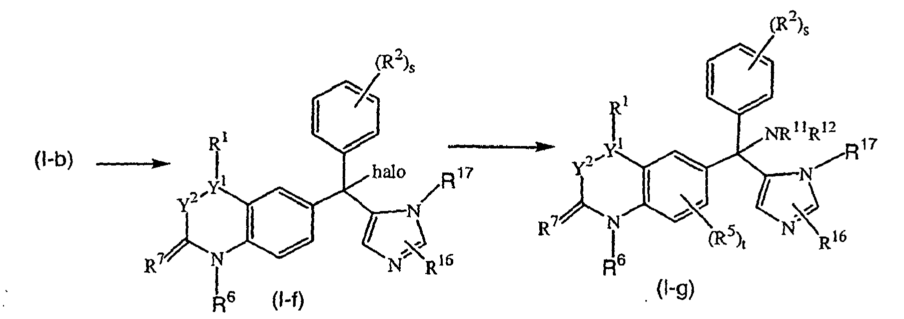
c) compounds of formula (I-b) can be converted into compounds of formula (I-g) for example by treatment with SOCl₂, and then NH₃/iPrOH, e.g. in a tetrahydrofuran solvent, or by treatment with acetic acid ammonium salt at a temperature ranging from 120 to 180°C, or by treatment with sulfamide at a temperature ranging from 120 to 180°C;
d) compounds of formula (I-f) can be converted into compounds of formula (I-c) for example by treatment with SnCl₂ in the presence of concentrated HCl in acetic acid at reflux;
e) compounds of formula (I) in which >Y¹-Y² represents a radical of formula (y-1) or (y-2) can be converted into corresponding compounds of formula (I) in which >Y¹-Y² represents a radical of formula (y-3) or (y-4) respectively, by conventional reduction procedures for example hydrogenation or reduction by treatment with sodium borohydride in a suitable solvent, e.g. methanol and *vice versa* by conventional oxidation procedures; e.g. oxidation with MnO₂ in a reaction-inert solvent, e.g. dichloromethane;
f) compounds of formula (I) in which X is oxygen can be converted into corresponding compounds of formula (I) in which X is sulphur with a reagent such as phosphorus pentasulfide or Lawesson's reagent in a suitable solvent such as, for example, pyridine;
g) compounds of formula (I) in which R⁹ is C₁₋₆alkyloxycarbonyl can be converted into corresponding compounds of formula (I) in which R⁹ is hydroxymethyl by conventional reduction procedures for example with the use of lithium aluminium hydride;
h) compounds of formula (I) in which R³ is a radical of formula (b-1) in which R¹⁰ is hydrogen can be converted into corresponding compounds of formula (I) in which R³ is a radical of formula (b-3) in which R¹¹ is hydrogen and R¹² is C₁₋₆alkylcarbonyl for example by with an appropriate nitrile for example acetonitrile.
i) compounds of formula (I) in which R⁶ is hydrogen can be converted into corresponding compounds of formula (I) in which R⁶ is C₁₋₆alkyl for example by treatment with an appropriate alkylating agent, e.g. a C₁₋₆ alkyl halide in the presence of a base for example NaH in an appropriate solvent such as THF or DMF.

The compounds of formula (I) may also be converted into each other via art-known reactions or functional group transformations. A number of such transformations are already described hereinabove. Other examples are hydrolysis of carboxylic esters to the corresponding carboxylic acid or alcohol; hydrolysis of amides to the corresponding carboxylic acids or amines; hydrolysis of nitriles to the corresponding amides; amino groups on imidazole or phenyl may be replaced by a hydrogen by art-known diazotation reactions and subsequent replacement of the diazo-group by hydrogen; alcohols may be converted into esters and ethers; primary amines may be converted into secondary or tertiary amines; double bonds may be hydrogenated to the corresponding single bond.

The intermediates and starting materials used in the above-described processes may be prepared in conventional manner using procedures known in the art for example as described in the above-mentioned patent specifications WO 97/16443, WO 97/21701, WO 98/40383, WO 98/49157 and WO 00/39082.

Thus compounds of formula (III) in which W¹ is chloro, used as starting materials in process b), can be prepared, for example in the case where R³ is hydroxy and R⁴ is a radical of formula (c-2) or (c-3), by reacting a compound of formula (VII): 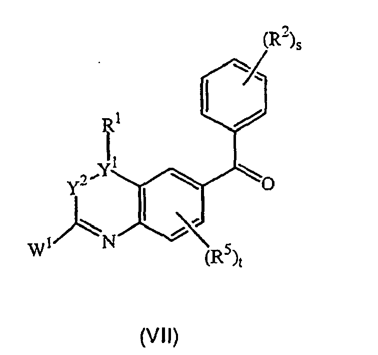 with an imidazole or triazole reagent in an analogous manner to that described above for process d).

The compound of formula (VII) can be prepared by chlorinating a compound of formula (VIII): 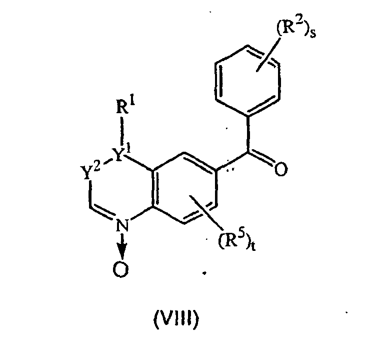 The chlorination of the above compound of formula (VII) can be conveniently effected by treatment with phosphorus oxychloride.

The compound of formula (VIII) can be prepared by oxidising a compound of formula (IX):

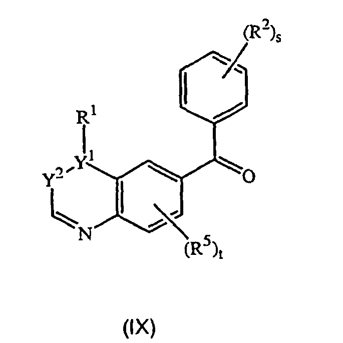

The oxidation of the compound of formula (IX) can be effected for example by treatment of the compound with a per-acid such as 3-chloro-benzenecarboperoxoic acid preferably in a reaction-inert solvent such as dichloromethane.

The compound of formula (IX) can be prepared for example from a compound of formula (X): 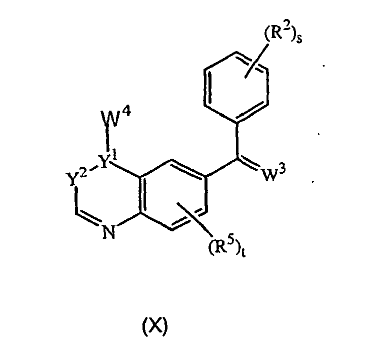 in which W³ is an oxo group or a protected oxo group such as an ethylenedioxy group and W⁴ is a leaving group or a precursor group for the R¹ moiety:

When W⁴ is a leaving group, this can be for example a halo e.g. chloro group which can be reacted with a compound of formula:

R¹-W⁵

in which W⁵ is a suitable leaving group: for example when Z in R¹ is -O- , the leaving group W⁵ can be hydrogen, and when W⁴ is chloro, the reaction can be conducted in the presence of sodium hydride preferably in a solvent such as dimethylformamide. Alternatively, when W⁴ is chloro and Z in R¹ is a bond, the W⁵ leaving group can be -B(OH)₂, the reaction being conveniently effected with triphenylphosphine-palladium reagent in the presence of a base such as potassium carbonate and in a suitable solvent such as DME.

When W⁴ is a precursor group, this can be for example a methyl group, which can be reacted with a compound of formula:

Het²-CH₂-W⁶

in which W⁶ is a suitable leaving group such as a halo (e.g. chloro) group to form a compound of formula (I) in which Z is a -CH₂CH₂- group; the reaction is advantageously effected in a basic medium for example comprising N-(1-methylethyl)-2-propanamine and N,N,N',N'-tetramethyl -1,2-ethanediamine with butyl lithium in a solvent such as tetrahydrofuran.

The oxo protection can be removed following the reaction of the compound of formula (X) for example by treatment with an acid such as hydrochloric acid in a solvent such methanol. Alternatively, the oxo protection can be retained to form corresponding oxo-protected forms of the compounds of formulae (VII) or (VIII), such protection being removed after the respective formation of such compounds; the removal may be effected in an analogous manner to that described for the conversion of compounds of formula (X) to compounds of formula (IX).

The compounds of formula (X) used as intermediates above can be prepared in conventional manner. Thus, when W⁴ is a methyl group, the compound can be prepared by reacting a compound of formula (XI): 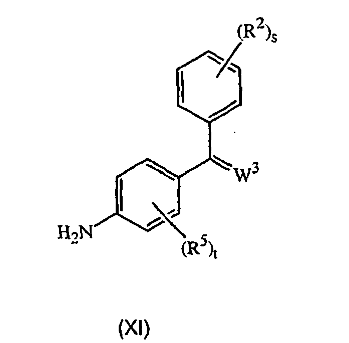 with an acid such as hydrochloric acid, then with FeCl₃ and ZnCl₂ before the addition of 3-buten-2-one. Compounds of formula (X) in which W⁴ is a different C₁₋₆alkyl group can be obtained in analogous manner. Compounds of formula (X) in which W⁴ is a chloro group can be obtained chlorination of the corresponding hydroxy group in a compound of formula (XII):
Compounds of formula (XII) can be prepared by cyclisation of compounds of formula (XI) in conventional manner.

Compounds of formula (III) in which W¹ is a C₁₋₆alkyloxy group and R³ is hydroxy can alternatively be prepared for example by reacting a compound of formula (XIII): 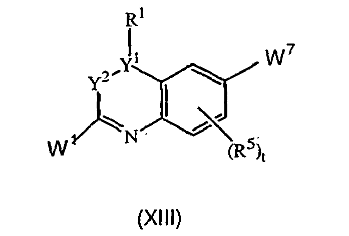 in which W⁷ is a leaving group for example a halo, e.g. bromo group, with a compound of formula (XIV):

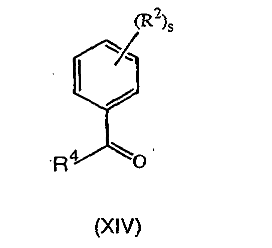

The reaction of the compounds of formulae (XIII) and (XIV) can be conveniently effected in the presence of n-butyl lithium, e.g. in solvent such as tetrahydrofuran.

The above compound of formula (XIII) can be prepared from a corresponding quinolinone compound for example by treatment with POCl₃ to form the corresponding 2-chloro compound which can then be reacted with an appropriate C₁₋₆alkanol to form the desired 2-C₁₋₆alkoxy compound. The starting quinolinone compound can be obtained by a cyclisation reaction as described in the Examples below.

The compounds of formula (IV) used as starting materials in process c) above for example in which R³ is hydrogen and W² is hydroxy can be prepared by reduction of corresponding compounds of formula (V), used as starting materials for process d); the reduction is conveniently effected by sodium borohydride in a solvent such as methanol. The corresponding compounds of formula (IV) in which W² is halo for example chloro can be obtained by halogenating the former hydroxy compounds, e.g. with thionyl chloride.

The compounds of formula (V) used as starting materials in process d) can be prepared for example by treatment of a compound of formula (VIII) above in an analogous manner to that described for process e) for example by reaction with tosyl chloride and subsequent hydrolysis of the resulting tosylate. If desired the resulting compound in which R⁶ is hydrogen can be converted to a compound with a different R⁶ group as described above.

Alternatively, compounds of formula (V), in which R¹ is Het² and Z is a bond, can be obtained by cyclisation of a compound of formula (XV): 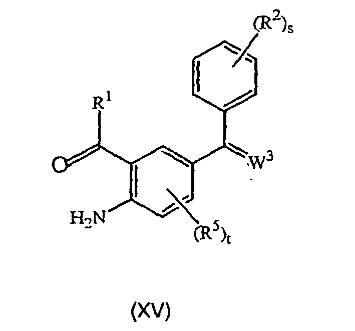 and if necessary removing the W³ oxo protection.

The cyclisation of the compound of formula (XV) can be effected in conventional manner for example using procedures analogous to those described in WO 97/16443, advantageously by subjecting the compound of formula (XV) to an acetylation reaction, e.g. by treatment with acetic anhydride in a reaction-inert solvent, e.g. toluene, optionally in the presence of a base to capture acid liberated during the reaction, and subsequent treatment with a base such potassium tert-butoxide in a reaction-inert solvent, e.g. 1,2-dimethoxyethane. The W³ oxo protected group can be converted to the free oxo group in conventional manner, for example as described above.

Compounds of formula (XV) can be obtained in conventional manner analogous to those described in WO 97/16443, from a compound of formula (XVI):

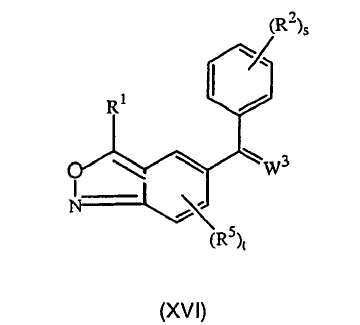

The compound of formula (XVI) can be reduced to a compound of formula (XV) for example using TiCl₃ in a reaction-inert solvent such as tetrahydrofuran.

The compound of formula (XVI) above can be obtained by reacting a compound of formula (XVII): 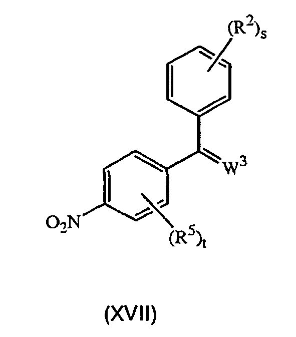 with a compound of formula (XVIII):

R¹-CH₂CN (XVIII)

for example using basic conditions such as sodium hydroxide in methanol.

Alternatively compounds of formula (V) in which Z is a bond can be prepared by cyclisation of a precursor compound of formula (XIX): 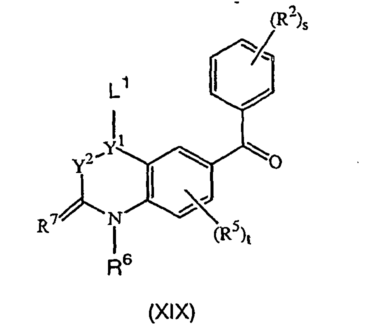 in which L¹ is a group which can be cyclised or which can be reacted with a reagent to form the Het² ring.

Thus for example, a compound of formula (XIX) in which L¹ is a -C(=S)NH₂ group, i.e. a compound of formula (XLX-a) can be reacted with CH₃COCH₂Cl to form a compound of formula (V) in which R¹ is a 4-methyl-1,3-thiazol-2-yl group. Other heterocyclic R1 groups can be constructed in an analogous manner.

The above compound of formula (XIX-a) can be prepared from the corresponding 4-methyl compound of formula (XX):

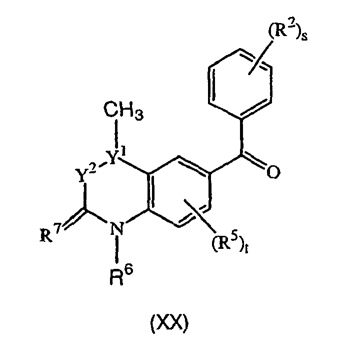

The above compound of formula (XX) can be converted into the desired compound of formula (XIX-a) by the following series of conventional transformations:
a) oxidation of the 4-methyl compound of formula (XX), e.g. with SeO₂ to form the corresponding 4-aldehyde compound;
b) oxidation of the 4-aldehyde compound, e.g. with KMnO₄ to form the corresponding 4-carboxyl compound;
d) chlorination of the 4-carboxyl compound, e.g. with SOCl₂ to form the corresponding 4-carbonyl chloride compound;
e) reaction of the 4-carbonyl chloride compound with ammonia to form the corresponding 4-CONH₂ compound;
f) reaction of the 4-CONH₂ compound with phosphorus oxychloride to form the corresponding 4-cyano compound;
g) reaction of the 4-cyano compound with pyridine to form the corresponding 4-C(=S)NH₂ compound of formula (XIX-a).

The above sequence of transformations can also include other reactions for example transformation of the R⁶ group, protection of the 4-carboxy group, e.g. with an ester group, or the use of corresponding quinoline compounds which can be converted into the corresponding quinolinone compounds (in which R⁷ is oxygen) for example as described above.

The above 4-carbonyl chloride compound can also be used to prepare a corresponding 4-oxadiazolyl compound as follows: thus the said 4-carbonylchloride compound can be reacted with (CH₃)₂NH₂CCH₂OH to form a corresponding 4-CONHC(CH₃)₂CH₂OH compound which can be chlorinated e.g. with thionyl chloride to form the 4-CONHC(CH₃)₂CH₂Cl compound which can then be cyclised , preferably under basic conditions, e.g. using sodium methoxide to form a 4,4-dimethyl-1,3-oxadiazol-2-yl compound of formula (1). Other analogous compounds of formula (I) can be prepared by appropriate analogous procedures.

The compounds of formula (I) and some of the intermediates have at least one stereogenic center in their structure. This stereogenic center may be present in a R or a S configuration.

The compounds of formula (I) as prepared in the hereinabove described processes are generally racemic mixtures of enantiomers which can be separated from one another following art-known resolution procedures. The racemic compounds of formula (I) may be converted into the corresponding diastereomeric salt forms by reaction with a suitable chiral acid. Said diastereomeric salt forms are subsequently separated, for example, by selective or fractional crystallization and the enantiomers are liberated therefrom by alkali. An alternative manner of separating the enantiomeric forms of the compounds of formula (I) involves liquid chromatography using a chiral stationary phase. Said pure stereochemically isomeric forms may also be derived from the corresponding pure stereochemically isomeric forms of the appropriate starting materials, provided that the reaction occurs stereospecifically. Preferably if a specific stereoisomer is desired, said compound will be synthesized by stereospecific methods of preparation. These methods will advantageously employ enantiomerically pure starting materials.

The compounds of formula (I), the pharmaceutically acceptable acid addition salts and stereoisomeric forms thereof have valuable pharmacological properties in that they have a potent farnesyl protein transferase (FPTase) inhibitory effect.

This invention provides the use of compounds of the invention in the manufacture of a medicament for inhibiting the abnormal growth of cells, including transformed cells, by administering an effective amount of a compound of the invention. Abnormal growth of cells refers to cell growth independent of normal regulatory mechanisms (e.g. loss of contact inhibition). This includes the abnormal growth of : (1) tumor cells (tumors) expressing an activated *ras* oncogene; (2) tumor cells in which the *ras* protein is activated as a result of oncogenic mutation of another gene; (3) benign and malignant cells of other proliferative diseases in which aberrant *ras* activation occurs. Furthermore, it has been suggested in literature that *ras* oncogenes not only contribute to the growth of tumors *in vivo* by a direct effect on tumor cell growth but also indirectly, *i*.*e*. by facilitating tumor-induced angiogenesis (Rak. J. et al, *Cancer Research,* 55, 4575-4580, 1995). Hence, pharmacologically targeting mutant *ras* oncogenes could conceivably suppress solid tumor growth *in vivo,* in part, by inhibiting tumor-induced angiogenesis.

This invention also provides the use of compounds of the invention in the manufacture of a medicament for inhibiting tumor growth by administering an effective amount of a compound of the present invention, to a subject, e.g. a mammal (and more particularly a human) in need of such treatment. In particular, this invention provides a method for inhibiting the growth of tumors expressing an activated *ras* oncogene by the administration of an effective amount of the compounds of the present invention. Examples of tumors which may be inhibited, but are not limited to, lung cancer (e.g. adenocarcinoma and including non-small cell lung cancer), pancreatic cancers (e.g. pancreatic carcinoma such as, for example exocrine pancreatic carcinoma), colon cancers (e.g. colorectal carcinomas, such as, for example, colon adenocarcinoma and colon adenoma), prostate cancer including the advanced disease, hematopoietic tumors of lymphoid lineage (e.g. acute lymphocytic leukemia, B-cell lymphoma, Burkitt's lymphoma), myeloid leukemias (for example, acute myelogenous leukemia (AML)), thyroid follicular cancer, myelodysplastic syndrome (MDS), tumors of mesenchymal origin (e.g. fibrosarcomas and rhabdomyosarcomas), melanomas, teratocarcinomas, neuroblastomas, gliomas, benign tumor of the skin (e.g. keratoacanthomas), breast carcinoma (e.g. advanced breast cancer), kidney carcinoma, ovary carcinoma, bladder carcinoma and epidermal carcinoma.

This invention may also provide a method for inhibiting proliferative diseases, both benign and malignant, wherein *ras* proteins are aberrantly activated as a result of oncogenic mutation in genes. With said inhibition being accomplished by the administration of an effective amount of the compounds described herein, to a subject in need of such a treatment. For example, the benign proliferative disorder neuro-fibromatosis, or tumors in which *ras* is activated due to mutation or overexpression of tyrosine kinase oncogenes, may be inhibited by the compounds of this invention.

The compounds of present invention may be useful for the treatment of proliferative diseases, both benign and malignant, wherein the K*-ras* B isoform is activated as a result of oncogenic mutation.

The compound according to the invention can be used for other therapeutic purposes, for example:
a) the sensitisation of tumors to radiotherapy by administering the compound according to the invention before, during or after irradiation of the tumor for treating cancer, for example as described in WO 00/01411;
b) treating athropathies such as rheumatoid arthritis, osteoarthritis, juvenile arthritis, gout, polyarthritis, psoriatic arthritis, ankylosing spondylitis and systemic lupus erythematosus, for example as described in WO 00/01386;
c) inhibiting smooth muscle cell proliferation including vascular proliferative disorders, atherosclerosis and restenosis, for example as described in WO 98/55124;
d) treating inflammatory conditions such as ulcerative colitis, Crohn's disease, allergic rhinitis, graft vs host disease, conjunctivitis, asthma, ARDS, Behcets disease, transplant rejection, uticaria, allergic dermatitis, alopecia areata, scleroderma, exanthem, eczema, dermatomyositis, acne, diabetes, systemic lupus erythematosis, Kawasaki's disease, multiple sclerosis, emphysema, cystic fibrosis and chronic bronchitis;
e) treating endometriosis, uterine fibroids, dysfunctional uterine bleeding and endometrial hyperplasia;
f) treating ocular vascularisation including vasculopathy affecting retinal and choroidal vessels;
g) treating pathologies resulting from heterotrimeric G protein membrane fixation including diseases related to following biological functions or disorders; smell, taste, light, perception, neurotransmission, neurodegeneration, endocrine and exocrine gland functioning, autocrine and paracrine regulation, blood pressure, embryogenesis, viral infections, immunological functions, diabetes, obesity;
h) inhibiting viral morphogenesis for example by inhibiting the prenylation or the post-prenylation reactions of a viral protein such as the large delta antigen of hepatitis D virus; and the treatment of HIV infections;
i) treating polycystic kidney disease;
j) suppressing induction of inducible nitric oxide including nitric oxide or cytokine mediated disorders, septic shock, inhibiting apoptosis and inhibiting nitric oxide cytotoxicity;
k) treating malaria.

Hence, the present invention discloses the compounds of formula (I) for use as a medicine as well as the use of these compounds of formula (I) for the manufacture of a medicament for treating one or more of the above-mentioned conditions.

For the treatment of the above conditions, the compound of the invention may be advantageously employed in combination with one or more other medicinal agents such as anti-cancer agents for example selected from platinum coordination compounds for example cisplatin or carboplatin, taxane compounds for example paclitaxel or docetaxel, camptothecin compounds for example irinotecan or topotecan, anti-tumor vinca alkaloids for example vinblastine, vincristine or vinorelbine, anti-tumor nucleoside derivatives for example 5-fluorouracil, gemcitabine or capecitabine, nitrogen mustard or nitrosourea alkylating agents for example cyclophosphamide, chlorambucil, carmustine or lomustine, anti-tumor anthracycline derivatives for example daunorubicin, doxorubicin or idarubicin; HER2 antibodies for example trastzumab; and anti-tumor podophyllotoxin derivatives for example etoposide or teniposide; and antiestrogen agents including estrogen receptor antagonists or selective estrogen receptor modulators preferably tamoxifen, or alternatively toremifene, droloxifene, faslodex and raloxifene, or aromatase inhibitors such as exemestane, anastrozole, letrazole and vorozole.

For the treatment of cancer the compounds according to the present invention can administered to a patient as described above in conjunction with irradiation; such treatment is may be especially beneficial as farnesyl transferase inhibitors can act as radiosensitisers for example as described in International Patent Specification WO 00/01411, enhancing the therapeutic effect of such irradiation.

Irradiation means ionizing radiation and in particular gamma radiation, especially that emitted by linear accelerators or by radionuclides that are in common use today. The irradiation of the tumor by radionuclides can be external or internal.

Preferably, the administration of the farnesyl transferase inhibitor commences up to one month, in particular up to 10 days or a week, before the irradiation of the tumor. Additionally, it is advantageous to fractionate the irradiation of the tumor and maintain the administration of the farnesyl transferase inhibitor in the interval between the first and the last irradiation session.

The amount of farnesyl protein transferase inhibitor, the dose of irradiation and the intermittence of the irradiation doses will depend on a series of parameters such as the type of tumor, its location, the patients' reaction to chemo- or radiotherapy and ultimately is for the physician and radiologists to determine in each individual case.

The present invention also concerns a method of cancer therapy for a host harboring a tumor comprising the steps of
- administering a radiation-sensitizing effective amount of a farnesyl protein transferase inhibitor according to the invention before, during or after
- administering radiation to said host in the proximity to the tumor.

In view of their useful pharmacological properties, the subject compounds may be formulated into various pharmaceutical forms for administration purposes.

To prepare the pharmaceutical compositions of this invention, an effective amount of a particular compound, in base or acid addition salt form, as the active ingredient is combined in intimate admixture with a pharmaceutically acceptable carrier, which carrier may take a wide variety of forms depending on the form of preparation desired for administration. These pharmaceutical compositions are desirably in unitary dosage form suitable, preferably, for administration orally, rectally, percutaneously, or by parenteral injection. For example, in preparing the compositions in oral dosage form, any of the usual pharmaceutical media may be employed, such as, for example, water, glycols, oils, alcohols and the like in the case of oral liquid preparations such as suspensions, syrups, elixirs and solutions; or solid carriers such as starches, sugars, kaolin, lubricants, binders, disintegrating agents and the like in the case of powders, pills, capsules and tablets.

Because of their ease in administration, tablets and capsules represent the most advantageous oral dosage unit form, in which case solid pharmaceutical carriers are obviously employed. For parenteral compositions, the carrier will usually comprise sterile water, at least in large part, though other ingredients, to aid solubility for example, may be included. Injectable solutions, for example, may be prepared in which the carrier comprises saline solution, glucose solution or a mixture of saline and glucose solution. Injectable suspensions may also be prepared in which case appropriate liquid carriers, suspending agents and the like may be employed. In the compositions suitable for percutaneous administration, the carrier optionally comprises a penetration enhancing agent and/or a suitable wetting agent, optionally combined with suitable additives of any nature in minor proportions, which additives do not cause a significant deleterious effect to the skin. Said additives may facilitate the administration to the skin and/or may be helpful for preparing the desired compositions. These compositions may be administered in various ways, e.g., as a transdermal patch, as a spot-on, as an ointment.

It is especially advantageous to formulate the aforementioned pharmaceutical compositions in dosage unit form for ease of administration and uniformity of dosage. Dosage unit form as used in the specification and claims herein refers to physically discrete units suitable as unitary dosages, each unit containing a predetermined quantity of active ingredient calculated to produce the desired therapeutic effect in association with the required pharmaceutical carrier. Examples of such dosage unit forms are tablets (including scored or coated tablets), capsules, pills, powder packets, wafers, injectable solutions or suspensions, teaspoonfuls, tablespoonfuls and the like, and segregated multiples thereof.

Those skilled in the art could easily determine the effective amount from the test results presented hereinafter. In general it is contemplated that an effective amount would be from 0.01 mg/kg to 100 mg/kg body weight, and in particular from 0.05 mg/kg to 10 mg/kg body weight. It may be appropriate to administer the required dose as two, three, four or more sub-doses at appropriate intervals throughout the day. Said sub-doses may be formulated as unit dosage forms, for example, containing 0.5 to 500 mg, and in particular 1 mg to 200 mg of active ingredient per unit dosage form.

The following examples are provided for purposes of illustration.

### Experimental part

Hereinafter "THF' means tetrahydrofuran, "DIPE" means diisopropyl ether, "DME" means 1,2-dimethoxyethane, "EtOAc" means ethyl acetate, "DCM" means dichloromethane, "DMF" means dimethylformamide and "BuLi" means n-butyl lithium.

### A. Preparation of the intermediates.

### Example A1

a) (4-Aminophenyl)(4-chlorophenyl)-methanone (0.104 mol) and diethyl (ethoxymethylene) malonate (0.114 mol) were stirred and heated at 130°C overnight. The product was used without further purification, yielding ethyl 2-[[[4-(4-chlorobenzoyl)phenyl]amino]carbonyl]-3-ethoxy-2-propenoate (intermediate 1).
b) A mixture of intermediate 1 (0.104 mol) in 1-1'-oxybis-benzene (100ml) was stirred and heated at 300°C for 8h. The mixture was taken up in diethyl ether and the product was filtered off, yielding 14.6g (39.5%) of ethyl 6-(4-chlorobenzoyl)-4-hydroxy-3-quinolinecarboxylate, melting point > 300°C (intermediate 2).
c) A mixture of intermediate 2 (0.051 mol) in sodium hydroxide (35ml) and water (100ml) was stirred and refluxed overnight. The mixture was cooled and poured onto water. The pH of the mixture was brought to 7 by adding HCl 6N and the mixture was filtered off. The precipitate was washed with diethyl ether, taken up in DCM and filtered off. The product was used without further purification, yielding 16g of 6-(4-chlorobenzoyl)-4-hydroxy-3-quinolinecarboxylic acid (intermediate 3).
d) Intermediate 3 (0.039 mol), Cu powder (0.031 mol) and quinoline (0.465 mol) were stirred at 250°C for 1h15min. The mixture was cooled, taken up in DCM and washed with water. The organic layer was dried (MgSO₄), filtered off and evaporated till dryness. The residue was taken up in diethyl ether, filtered off and dried, yielding 7.9g (71%) of (4-chlorophenyl) (4-hydroxy-6-quinolinyl)methanone (intermediate 4).
e) A mixture of intermediate 4 (0.027 mol) in phosphoryl chloride (100ml) was stirred at 60°C for 3h. The mixture was evaporated, the residue was taken up in DCM and basified with K₂CO₃ 10%. The organic layer was dried (MgSO₄), filtered off and evaporated. The product was used without further purification, yielding 7.6g (91%) of (4-chlorophenyl)(4-chloro-6-quinolinyl)methanone (intermediate5).
f) 3-Chloro-benzenecarboperoxoic acid (0.042 mol) was added at room temperature to a solution of intermediate 5 (0.021 mol) in DCM (60ml) and the mixture was stirred at room temperature for one night. K₂CO₃ 10% and DCM were added, the organic layer was decanted, dried (MgSO₄), filtered off and evaporated till a volume of 100ml which was used without further purification, yielding (quant.) of (4-chloro-1-oxido-6-quinolinyl)(4-chlorophenyl)- methanone (intermediate 6).
g) 4-Methyl-benzenesulfonyl chloride (0.027 mol) was added portionwise at room temperature to a solution of intermediate 6 (0.021 mol) in K₂CO₃10% (80ml) and DCM (100ml) and the mixture was stirred at room temperature for 1h. The mixture was evaporated, the precipitate was filtered off, washed with water, then with diethyl ether and air-dried. The product was used without further purification, yielding 6.6g (99%) of 4-chloro-6-(4-chlorobenzoyl)- 2(1*H*)-quinolinone (intermediate 7).
h) Intermediate 7 (0.0189 mol) and 1-*H*-imidazole (0.113 mol) were stirred at 120°C for 24h. The mixture was taken up in DCM and methanol and washed with K₂CO₃ 10%. The organic layer was dried (MgSO₄), filtered off and evaporated till dryness. The residue was purified by column chromatography over silica gel (eluent : CH₂Cl₂/CH₃OH/NH₄OH 95/5/0.1). The pure fractions were collected and evaporated, yielding 2.3g (34%) of 6-(4-chlorobenzoyl)-4-(1*H*-imidazol-1-yl)- 2(1*H*)-quinolinone (intermediate 8).
i) Sodium tetrahydroborate (0.00571 mol) was added portionwise at 5°C to a solution of intermediate 8 (0.00571 mol) in methanol (20ml) and THF (20ml) and the mixture was stirred at 5°C for 30min. The mixture was poured into water, the precipitate was filtered off and dried, yielding 1.8g (85%) of 6-[(4-chlorophenyl)hydroxymethyl]-4-(1*H*-imidazol-1-yl)- 2(1*H*)-quinolinone (intermediate 9).
j) A mixture of intermediate 9 (0.00483 mol) in thionyl chloride (50ml) was stirred at room temperature for 12h. The mixture was evaporated till dryness and the product was used without further purification, yielding 1.8g (quant.) of 6-[chloro(4-chlorophenyl)methyl]-4-(1*H*-imidazol-1-yl)- 2(1*H*)-quinolinone (intermediate 10).

### Example A2

a) 2-Thiopheneacetonitrile (0.147 mol) and then.2-(4-chlorophenyl)-2-(4-nitrophenyl)-1,3-dioxolane (0.0818 mol) were added to a solution of sodium hydroxide (0.327 mol) in methanol (100ml). The mixture was stirred at 60°C for 4 hours. Ice water was added. The mixture was extracted with DCM and decanted. The organic layer was dried (MgSO₄), filtered and the solvent was evaporated. The residue (92g) was purified by column chromatography over silica gel (eluent: DCM; 20-45 µm). The pure fractions were collected and the solvent was evaporated, yielding 9g (29%) of 5-[2-(4-chlorophenyl)-1,3-dioxolan-2-yl]-3-(2-thienyl)- 2,1-benzisoxazole (intermediate 11).
b) A mixture of intermediate 11 (0.03 mol) in Ti Cl₃ 15% in water (60ml) and THF (60ml) was stirred at room temperature for the weekend. Water was added. The mixture was extracted with DCM. The organic layer was separated, washed with K₂CO₃ 10% and decanted. The organic layer was dried (MgSO₄) filtered and the solvent was evaporated, yielding 10g (98%) of [2-amino-5-(4-chlorobenzoyl)phenyl]-2-thienyl-methanone (intermediate 12).
c) A mixture of intermediate 12 (0.029 mol) and acetic acid anhydride (0.058 mol) in toluene (150ml) was stirred at 120°C for 15 hours. The solvent was evaporated. The product was used without further purification, yielding N-[4-(4-chlorobenzoyl)-2-(2-thienylcarbonyl)phenyl]- acetamide (intermediate 13).
d) A mixture of intermediate 13 (0.029 mol) and 2-methyl-2-propanol, potassium salt (0.116 mol) in 1,2-dimethoxyethane (110ml) was stirred at room temperature overnight. Water was added. The solvent was evaporated. The product was used without further purification, yielding 6-(4-chlorobenzoyl)-4-(2-thienyl)- 2(1*H*)-quinolinone (intermediate 14).
e) Benzyltriethylammonium chloride (0.0029 mol) and then iodomethane (0.058 mol) were added to a mixture of intermediate 14 (0.029 mol) in THF (100ml) and concentrated sodium hydroxide (100ml). The mixture was stirred at room temperature overnight. Water was added. The mixture was extracted with DCM. The organic layer was separated, dried (MgSO₄), filtered and the solvent was evaporated. The residue was crystallized from CH₃CN. The precipitate was filtered off, washed with DIPE and dried, yielding 7.3g (66%) of 6-(4-chlorobenzoyl)-1-methyl-4-(2-thienyl)- 2(1*H*)-quinolinone (intermediate 15).

### Example A3

a) BuLi 1.6 M in hexane (0.165 mol) was added at -78°C to 3-bromo-pyridine (0.15 mol) dissolved in diethyl ether (850ml) and the mixture was stirred at -78°C for 30min. 6-Bromo-2-methyl-4*H*-3,1-benzoxazin-4-one (0.15 mol) was added portionwise and the mixture was brought till -20°C. The mixture was hydrolyzed, decanted and extracted with EtOAc. The organic layer was dried (MgSO₄), filtered off and evaporated to dryness. The residue (41.1g) was purified by column chromatography over silica gel (eluent: CH₂Cl₂/CH₃OH/NH₃OH 99.5/0.5/0.1) (35-70µm). The pure fractions were collected and evaporated, yielding 15g (31%) of *N*-[4-bromo-2-(3-pyridinylcarbonyl)phenyl]-acetamide (intermediate 16).
b) 2-Methyl-2-propanol, potassium salt (0.188 mol) was added to intermediate 16 (0.0476 mol) dissolved in 1,2-dimethoxyethane (150ml) and the mixture was stirred at room temperature for one night. The mixture was hydrolysed and evaporated. The residue was taken up in DCM and ethanol and decanted. The organic layer was dried (MgSO₄), filtered off and evaporated till dryness, yielding 12.3g (89%) of 6-bromo-4-(3-pyridinyl)- 2(1*H*)-quinolinone (intermediate 17).
c) A mixture of intermediate 17 (0.0445 mol) in phosphoryl chloride (135ml) was stirred and heated at 40°C for one night. The mixture was evaporated till dryness, yielding 15.8g (quant.) of 6-bromo-2-chloro-4-(3-pyridinyl)- quinoline hydrochloride (1:1) (intermediate 18).
d) CH₃ONa 30% /CH₃OH (250ml) was added to a solution of intermediate 18 (0.0445 mol) in methanol (100ml) and the mixture was stirred and heated at 60°C for one night. The mixture was poured into ice, water was added and extracted with DCM. The organic layer was dried (MgSO₄) filtered off and evaporated till dryness. The residue was crystallized from diethyl ether and DIPE, filtered off and dried. The residue was purified by column chromatography over silica gel (eluent: CH₂Cl₂/CH₃OH/NH₄OH 99/1/0.1) (15-40*µ*m). The pure fractions were collected and evaporated, yielding 3.2g (23%) of 6-bromo-2-methoxy-4-(3-pyridinyl)- quinoline (intermediate 19).
e) BuLi 1.6M in hexane (0.0109 mol) was added dropwise at -70°C to a mixture of intermediate 19 (0.0073 mol) in THF (40ml). The mixture was stirred at -70°C for 30 min. A mixture of (4-chlorophenyl)(1-methyl-1*H*-imidazol-5-yl)-methanone (0.0109 mol) in THF (50ml) was added dropwise. The mixture was stirred at -70°C for 2 hours, hydrolyzed and extracted with DCM. The organic layer was separated, dried (MgSO₄), filtered and the solvent was evaporated till dryness. The residue (4.84g) was purified by column chromatography over silica gel (eluent: CH₂Cl₂/CH₃OH/NH₄OH 94/6/0.2; 15-40 µm). The pure fractions were collected and the solvent was evaporated, yielding 1.2g (36%) of α-(4-chlorophenyl)-2-methoxy-α-(1-methyl-1*H*-imidazol-5-yl)-4-(3-pyridinyl)- 6-quinolinemethanol (intermediate 20).

### Example A4

a) HCl/diethyl ether (0.15 mol) was added dropwise to a solution of (4-aminophenyl)(4-chlorophenyl)-methanone (0.15 mol) in ethanol (250ml) and the mixture was stirred for 15min. FeCl₃.6 H₂O (0.255 mol) and then ZnCl₂ (0.015 mol) were added and the mixture was stirred at 65°C for 30min. 3-Buten-2-one (0.15 mol) was added and the mixture was stirred at 80°C overnight. The mixture was poured into ice and basified with NH₄OH. The suspension was filtered through celite and the filtrate was extracted with DCM. The organic layer was dried (MgSO₄) filtered off and evaporated till dryness. The product was used without further purification, yielding : 43.1g (100%) of (4-chlorophenyl)(4-methyl-6-quinolinyl)- methanone, melting point 114 °C (intermediate 21).
b) A mixture of intermediate 21 (0.177 mol) and SeO₂ (0.177 mol) in dioxane (160ml) and water (38ml) was stirred and refluxed overnight and then cooled. DCM was added. The mixture was filtered over celite, dried (MgSO₄), filtered and the solvent was evaporated till dryness, yielding 60.9g (>100%) of 6-(4-chlorobenzoyl)- 4-quinolinecarboxaldehyde (intermediate 22).
c) A solution of KMnO₄ (0.23 mol) in water (500ml) was added to a mixture of intermediate 22 (0.177 mol) in 2-propanone (1400ml). The mixture was stirred at room temperature overnight and then filtered over celite. 2-propanone was evaporated. The concentrate was neutralized with HCl 3N. The precipitate was filtered off, washed with water and dried, yielding: 44.8g (81%) of fraction 1. Part of this fraction 1 (1.5g) was crystallized from methanol and H2O. The precipitate was filtered off and dried, yielding 1.3g (87%) of 6-(4-chlorobenzoyl)- 4-quinolinecarboxylic acid (intermediate 23), melting point > 260°C.
d) Thionyl chloride (0.352 mol) was added dropwise at 0°C under N₂ flow to a mixture of intermediate 23 (0.131 mol) in methanol (400ml). The mixture was stirred at room temperature for 1 hour, at 80°C overnight and cooled. The solvent was evaporated. The residue was taken up in K₂CO₃ 10% and extracted with EtOAc. The organic layer was separated, dried (MgSO₄), filtered and the solvent was evaporated till dryness, yielding 30g (78%) of fraction 1. A part of this fraction 1 was purified by column chromatography over silica gel (eluent: CH₂Cl₂/EtOAc 95/5; 20-45 *µ*m). The pure fractions were collected and the solvent was evaporated. The residue was crystallized from 2-propanone and methanol. The precipitate was filtered off and dried, yielding 1.2g of 6-(4-chlorobenzoyl)- 4-quinolinecarboxylic acid, methyl ester (intermediate 24), melting point 126°C.
e) A mixture of intermediate 24 (0.0189 mol) and 3-chloro-benzenecarboperoxoic acid (0.0245 mol) in DCM (60ml) was stirred at room temperature for 3 hours. K₂CO₃ 10% was added and the mixture was extracted with DCM. The organic layer was separated, dried (MgSO₄), filtered and the solvent was evaporated till dryness, yielding: 8.5g (>100%) of 6-(4-chlorobenzoyl)-4-quinolinecarboxylic acid, methyl ester, 1-oxide (intermediate 25).
f) A mixture of intermediate 25 (0.0189 mol) in HCl 6N (80ml) and methanol (80ml) was stirred and refluxed for 18 hours. The mixture was hydrolyzed. The precipitate was filtered off, washed with diethyl ether and dried, yielding 5g (77%) of 6-(4-chlorobenzoyl)-1,2-dihydro-2-oxo-4-quinolinecarboxylic acid, methyl ester (intermediate 26).
g) Sodium hydride 80% in oil (0.0908 mol) was added portionwise under N₂ flow to a mixture of intermediate 26 (0.0568 mol) in DMF (200ml). The mixture was stirred at 60°C for 2 hours. Iodomethane (0.0908 mol) was added. The mixture was stirred at 60°C for 2 hours, then hydrolyzed, filtered and taken up in methanol. The solvent was evaporated. The residue was taken up in diethyl ether. The precipitate was filtered off and dried, yielding 17.3g (85%) of 6-(4-chlorobenzoyl)-1,2-dihydro-1-methyl-2-oxo-4-quinolinecarboxylic acid, methyl ester, melting point 182°C (intermediate 27).
h) A mixture of intermediate 27 (0.0464 mol) in concentrated sodium hydroxide (60ml) and methanol (120ml) was stirred at room temperature for 15 min. The mixture was poured out into ice water and acidified with HCl 3N. The precipitate was filtered off, washed with water and dried with methanol and diethyl ether, yielding 12.4g (78%) of 6-(4-chlorobenzoyl)-1,2-dihydro-1-methyl-2-oxo-4-quinolinecarboxylic acid, melting point >260 °C (intermediate 28).
i) A mixture of intermediate 28 (0.0664 mol) in thionyl chloride (200ml) was stirred and refluxed for a while. The solvent was evaporated till dryness. The product was used without further purification, yielding 6-(4-chlorobenzoyl)-1,2-dihydro-1-methyl-2-oxo-4-quinolinecarbonyl chloride (intermediate 29).
j) A solution of intermediate 29 (0.0246 mol) in THF (40ml) was added dropwise at 5°C to NH₃/2-propanol (40ml). The mixture was stirred at room temperature for the weekend. Water (20ml) and diethyl ether were added. The precipitate was filtered off and taken up in toluene. The solvent was evaporated till dryness, yielding 7.1 g (85%) of 6-(4-chlorobenzoyl)-1,2-dihydro-1-methyl-2-oxo-4-quinolinecarboxamide (intermediate 30).
k) A mixture of intermediate 30 (0.0208 mol) in phosphoryl chloride (50ml) was stirred and refluxed overnight. The solvent was evaporated till dryness. The residue was cooled on ice. Methanol was added. The precipitate was filtered off, washed with DIPE and dried, yielding 5.6g (83%) of 6-(4-chlorobenzoyl)-1,2-dihydro-1-methyl-2-oxo-4-quinolinecarbonitrile (intermediate 31).
1) H₂S was bubbled through a mixture of intermediate 31 (0.102 mol) and acetonitrile (0.102 mol) in pyridine (350ml) at 60°C for 4.5 hours. The solvent was evaporated till dryness. The residue was taken up in HCl 1N and the mixture was extracted with DCM (precipitation resulted). The solvent was evaporated till dryness. The residue was taken up in 2-propanone. The precipitate was filtered off, washed with 2-propanone and with diethyl ether and dried, yielding 31.5g (87%) of fraction 1. Part of this fraction 1 (1.2g) was crystallized from DCM and methanol. The precipitate was filtered off, washed with diethyl ether and dried, yielding 0.8g (67%) of 6-(4-chlorobenzoyl)-1,2-dihydro-1-methyl-2-oxo-4-quinolinecarbothioarnide (intermediate 32), melting point 210°C. m) A mixture of intermediate 32 (0.0119 mol) and 2-bromo-1-phenyl-ethanone (0.0131 mol) in ethanol (150ml) was stirred and refluxed for 2 hours and then cooled. DCM was added. The organic solution was washed with water, dried (MgSO₄), filtered and the solvent was evaporated till dryness. The residue (7.8g) was purified by column chromatography over silica gel (eluent: CH₂Cl₂/EtOAc 95/5 and 94/6; 20-45 µm). The pure fractions were collected and the solvent was evaporated, yielding 4g (74%) of 6-(4-chlorobenzoyl)-1-methyl-4-(4-phenyl-2-thiazolyl)- 2(1*H*)-quinolinone (intermediate 33), melting point 216°C.

### Example A5

A mixture of intermediate 32 (0.0224 mol) and 1-chloro-2-propanone (0.0247 mol) in ethanol (160ml) was stirred and refluxed overnight, then taken up in DCM and methanol and washed with K₂CO₃ 10%. The organic layer was separated, dried (MgSO₄) filtered and the solvent was evaporated till dryness. The residue (8g) was purified by column chromatography over silica gel (eluent: CH₂Cl₂/CH₃OH 98.5/1.5 and 98/2). The pure fractions were collected and the solvent was evaporated. The residue was crystallized from diethyl ether. The precipitate was filtered off, washed with diethyl ether and dried, yielding 2.3g (26%) of 6-(4-chlorobenzoyl)-1-methyl-4-(4-methyl-2-thiazolyl)- 2(1*H*)-quinolinone (intermediate 34), melting point 236°C.

### Example A6

a) NaBH₄ (0.0044 mol) was added at 0°C to a mixture of intermediate 33 (0.0044 mol) in methanol (20ml) and THF (20ml). The mixture was stirred at room temperature for 30 min, hydrolyzed and extracted with DCM. The organic layer was separated, dried (MgSO₄), filtered and the solvent was evaporated till dryness. The residue (2g) was purified by column chromatography over silica gel (eluent: CH₂Cl₂/CH₃OH 98/2; 15-40 µm). The pure fractions were collected and the solvent was evaporated. The residue was taken up in 2-propanone and diethyl ether. The precipitate was filtered off and dried, yielding 1.45g (72%) of 6-[(4-chlorophenyl)hydroxymethyl]-1-methyl-4-(4-phenyl-2-thiazolyl)- 2(1*H*)-quinolinone (intermediate 35), melting point 192°C.
b) A mixture of intermediate 35 (0.0048 mol) in thionyl chloride (20ml) was stirred at room temperature for 2 hours. The solvent was evaporated till dryness. The product was used without further purification, yielding 6-[chloro(4-chlorophenyl)methyl]-1-methyl-4-(4-phenyl-2-thiazolyl)- 2(1*H*)-quinolinone (intermediate 36).

### Example A7

a) A mixture of intermediate 21 (0.15 mol), 1,2 ethanediol (0.54 mol) and paratoluenesulfonic acid (0.18 mol) in toluene (600ml) was stirred and refluxed in a Dean Stark apparatus overnight. The mixture was cooled, basified with K₂CO₃ 10% and extracted with DCM. The organic layer was dried (MgSO₄), filtered off and evaporated till dryness. The residue was purified by column chromatography over silica gel (eluent: CH₂Cl₂/CH₃OH/NH₄OH 99.75/0.25/0.2) (35-70µm). The pure fractions were collected and evaporated, yielding 35g (73%) of 6-[2-(4-chlorophenyl)-1,3-dioxolan-2-yl]-4.-methyl-quinoline (intermediate 37).
b) BuLi (0.0239 mol) was added dropwise at -20°C to a mixture of N (1-methylethyl)-2-propanamine HCl (0.0239 mol) and N,N,N',N'-tetramethylethylenediamine (0.0239 mol) in THF (10ml) under N₂ flow. The mixture was stirred at -20°C for 15 minutes. A solution of intermediate 37 (0.0184 mol) in THF (20ml) was added. The mixture was stirred at -20°C for 1 hour. 2,5-Bis(chloromethyl)-thiophene (0.0221 mol) was added. The mixture was brought to room temperature for 4 hours. Water was added. The mixture was extracted with EtOAc. The organic layer was separated, dried (MgSO₄), filtered, and the solvent was evaporated. The residue (10.2g) was purified by column chromatography over silica gel (eluent: CH₂Cl₂/CH₃OH 98.5/1.5; 15-40*µ*m). The pure fractions were collected and the solvent was evaporated. The residue (4.4g, 52%) was crystallized from diethyl ether. The precipitate was filtered off and dried, yielding 1.9g (23%) of 6-[2-(4-chlorophenyl)-1,3-dioxolan-2-yl]-4-[2-(5-chloro-2-thienyl)ethyl]-quinoline (intermediate 38), melting point 94°C.
c) m-Chloroperoxybenzoic acid (0.0083 mol) was added to a mixture of intermediate 38, (0.0041 mol) in DCM (19ml). The mixture was stirred at room temperature for 1 hour, poured out into K₂CO₃, extracted with DCM and washed with water. The organic layer was separated, dried (MgSO₄), filtered and the solvent was evaporated till 15ml of DCM left, yielding 6-[2-(4-chlorophenyl)-1,3-dioxolan-2-yl]-4-[2-(5-chloro-2-thienyl)ethyl]-quinoline, 1-oxide (intermediate 39). This product was used without further purification.
d) A mixture of intermediate 39 (0.0041 mol) and tosyl-chloride (0.0062 mol) in potassium carbonate (20ml) and DCM (20ml) was stirred at room temperature for 18 hours; poured out into water and extracted with DCM. The organic layer was separated, dried (MgSO₄), filtered and the solvent was evaporated. The residue was crystallized from CH₂Cl₂/diethyl ether. The precipitate was filtered off and dried, yielding 1g (51%) of 6-[2-(4-chlorophenyl)-1 ,3-dioxolan-2-yl]-4-[2-(5-chloro-2-thienyl)ethyl]- 2(1*H*)-quinolinone (intermediate 40).
e) BTEAC (0.00021 mol) then iodomethane (0.0042 mol) were added to a mixture of intermediate 40, (0.0021 mol) in NaOH (3ml) and THF (7ml). The mixture was stirred at room temperature for 4 hours. Water was added. The mixture was extracted with EtOAc. The organic layer was separated, dried (MgSO₄) filtered and the solvent was evaporated, yielding 1.1 g of 6-[2-(4-chlorophenyl)-1,3-dioxolan-2-yl]-4-[2-(5-chloro-2-thienyl)ethyl]-1-methyl-2(1*H*)-quinolinone (intermediate 41). This product was used without further purification.
f) A mixture of intermediate 41 (0.0032 mol) in HCl 3N (8ml) and THF (8ml) was stirred and refluxed for 18 hours, then cooled, poured out into water, basified with NH₄OH and extracted with EtOAc. The organic layer was separated, dried (MgSO₄), filtered and the solvent was evaporated. The residue (1.4g) was crystallized from 2-propanone. The precipitate was filtered off and dried, yielding 0.51g (35%) of 6-(4-chlorobenzoyl)-4-[2-(5-chloro-2-thienyl)ethyl]-1-methyl-2(1*H*)-quinolinone (intermediate 42), melting point 151°C.

### B. Preparation of the final compounds

### Example B1

A mixture of intermediate (10) (0.00483 mol) and 1*H*-imidazole (0.029 mol) in acetonitrile (100ml) was stirred and refluxed for 3h. The mixture was evaporated to dryness, the residue was taken up in water and filtered off. The oily residue was taken up in DCM, dried (MgSO₄), filtered off and evaporated to dryness. The residue was purified by column chromatography over silica gel (eluent : CH₂Cl₂/CH₃OH/NH₄OH 95/5/0.2) (15-40*µ*m). The pure fractions were collected and evaporated. The residue was recrystallized from diethyl ether and 2-propanone, yielding 0.6g (30%) of 6-[(4-chlorophenyl)-1*H*-imidazol-1-ylmethyl]-4-(1*H*-imidazol-1-yl)- 2(1*H*)-quinolinone, melting point 242.3°C.

### Example B2

BuLi (41ml) was added dropwise at -70°C under N₂ flow to a solution of 1-methyl-1H imidazole (0.066 mol) in THF (200ml). The mixture was stirred at -70°C for 45 min. Chlorotriethyl-silane (0.066 mol) was added and the mixture was allowed to warm to room temperature. The mixture was cooled to -70°C, BuLi (41ml) was added slowly and the mixture was stirred at -70°C for 1 hour. The mixture was brought to -15°C and cooled to -70°C again. Intermediate (15) (0.013 mol) was added and the mixture was brought to -10°C. Water was added. The mixture was extracted twice with EtOAc. The combined organic layer was dried (MgSO₄), filtered and the solvent was evaporated. The residue (18g) was purified by column chromatography over silica gel (eluent: CH₂Cl₂/CH₃OH/NH₄OH 96/4/0.1; 15-40 *µ*m). Two pure fractions were collected and their solvents were evaporated, yielding 2.1g F1 and 1.1g F2 (total yield 53%). F1 was crystallized from 2-propanone. The precipitate was filtered off and dried, yielding 1.7g (80%) of 6-[(4-chlorophenyl)hydroxy(1-methyl-1*H*-imidazol-5-yl)methyl]-1-methyl-4-(2-thienyl)- 2(1*H*)-quinolinone, melting point > 260°C.

### Example B3

A mixture of intermediate (20) (0.0026 mol) in HCl 3N (15ml) and THF (15ml) was stirred and refluxed overnight. The mixture was poured out on ice and basified with NH₄OH. The precipitate was filtered off, washed with water and dried with diethyl ether. The residue was crystallized from 2-propanone and methanol. The precipitate was filtered off, washed with diethyl ether and dried, yielding 0.75g (65%) of 6-[(4-chlorophenyl)hydroxy(1-methyl-1*H* imidazol-5-yl)methyl]-4-(3-pyridinyl)- 2(1*H*)-quinolinone, melting point >250°C.

### Example B4

BuLi 1.6M in hexane (6.6ml) was added dropwise at -70°C to a mixture of 1-methyl-1*H*-imidazole (0.0105 mol) in THF (30ml). The mixture was stirred for 30 min. Chlorotriethyl-silane (0.0105 mol) was added. The mixture was brought slowly to room temperature and cooled again to -70°C. BuLi 1.6M in hexane (6.6ml) was added dropwise. The mixture was stirred at -70°C for 1 hour, brought quickly to -15°C and cooled to -70°C. A suspension of intermediate (33) (0.0088 mol) in THF (40ml) was added dropwise. The mixture was brought to -20°C and hydrolyzed. The precipitate was filtered off. The filtrate was extracted with DCM. The organic layer was separated, dried (MgSO₄), filtered and the solvent was evaporated till dryness. The residue (4.9g) was purified by column chromatography over silica gel (eluent: CH₂Cl₂/CH₃OH/NH₄OH 96/4/0.1; 15-40 µm). The pure fractions were collected and the solvent was evaporated. The residue was taken up in DCM. The organic solution was washed several times with water, dried (MgSO₄), filtered and the solvent was evaporated till dryness. The residue was taken up in diethyl ether. The precipitate was filtered off and dried, yielding 0.6g (12%) of 6-[(4-chlorophenyl)hydroxy(1-methyl-1*H*-imidazol-5-yl)methyl]-1-methyl-4-(4-phenyl-2-thiazolyl)- 2(1*H*)-quinolinone, melting point 200°C.

### Example B5

BuLi (6.3ml) was added dropwise at -70°C to a mixture of 1-methyl-1*H*-imidazole (0.01 mol) in THF (35ml). The mixture was stirred for 30 min. Chlorotriethyl-silane (0.01 mol) was added dropwise. The mixture was brought slowly to 10°C and cooled again to -70°C. BuLi (6.3ml) was added dropwise. The mixture was stirred at -70°C for 1 hour, brought to -40°C and cooled again to -70°C. Intermediate (34) (0.0084 mol) was added portionwise. The mixture was brought to -50°C, hydrolyzed and extracted with DCM/methanol. The organic layer was separated, dried (MgSO₄), filtered and the solvent was evaporated to dryness. The residue (3.7g) was purified by column chromatography over silica gel (eluent: toluene/2-propanol/NH₄OH 85/15/1; 15-40 µm). Two pure fractions were collected and their solvents were evaporated, yielding 0.28g F1 (7%) and 1.15g F2 (35% starting material: intermediate 34). F1 was dissolved in 2-propanone and converted into the ethanedioic acid salt (1:1). The precipitate was filtered off and dried, yielding 0.3g (6%) of 6-[(4-chlorophenyl)hydroxy(1-methyl-1H-imidazol-5-yl)methyl]-1-methyl-4-(4-methyl-2-thiazolyl)- 2(1*H*)-quinolinone ethanedioate (1:1) hydrate (1:1), melting point 174°C.

### Example B6

A mixture of 6-[(4-chlorophenyl)hydroxy(1-methyl-1H-imidazol-5-yl)methyl]-1-methyl-4-(4-phenyl-2-thiazolyl)- 2(1*H*)-quinolinone (see Example B4) (0.0046 mol) in formamide (10ml) and acetic acid (20ml) was stirred at 160°C for 2 hours, poured out on ice, basified with NH₄OH and extracted with DCM. The organic layer was separated, dried (MgSO₄), filtered and the solvent was evaporated. The residue (2.9g) was purified by column chromatography over silica gel (eluent: CH₂Cl₂/CH₃OH/NH₄OH 97/3/0.1; 20-45 µm). The pure fractions were collected and the solvent was evaporated. The residue was crystallized from CH₃CN and DIPE. The precipitate was filtered off, washed with diethyl ether and dried, yielding 1.7g (70%) of 6-[(4-chlorophenyl)(1-methyl-1*H*-imidazol-5-yl)methyl]-1-methyl-4-(4-phenyl-2-thiazolyl)- 2(1*H*)-quinolinone, melting point 171°C.

### Example B7

a) Thionyl chloride (20 ml) was stirred at 5 °C. 6-[(4-chlorophenyl)hydroxy(1-methyl-1*H*-imidazol-5-yl)methyl]-1-methyl-4-(4-phenyl-2-thiazolyl)- 2(1*H*)-quinolinone (see Example B4) (0.0037 mol) was added portionwise and the reaction mixture was stirred for 6 hours. The solvent was evaporated, yielding crude residue (quantitative yield; used in next reaction step, without further purification) of 6-[chloro(4-chlorophenyl)(1-methyl-1*H*-imidazol-5-yl)methyl]-1-methyl-4-(4-phenyl-2-thiazolyl)- 2(1*H*)-quinolinone.
b) A solution of 6-[chloro(4-chlorophenyl)(1-methyl-1H-imidazol-5-yl)methyl]-1-methyl-4-(4-phenyl-2-thiazolyl)- 2(1*H*)-quinolinone (see Example B7a) (0.0037 mol) in THF (20 ml) was stirred at 5 °C. NH₃ 2.5N/2-propanol (10 ml) was added and the reaction mixture was stirred for 3 hours at 5 °C. The reaction mixture was hydrolyzed with 10% K₂CO₃, then extracted with DCM/methanol. The separated organic layer was dried (MgSO₄) filtered and the solvent evaporated. The residue (2.6 g) was purified by column chromatography over silica gel (450 g, 20-45 *µ*m; eluent: toluene/2-propanol/NH₄OH 85/15/0.2). Two pure fraction groups were collected and their solvent was evaporated to give 0.55 g (F1) and 0.76 g (F2). Fraction (F2) was recrystallized from acetonitrile/diethyl ether, filtered off and dried, yielding 0.70 g (35%) of 6-[amino(4-chlorophenyl)(1-methyl-1*H*-imidazol-5-yl)methyl]-1-methyl-4-(4-phenyl-2-thiazolyl)- 2(1*H*)-quinolinone, melting point 237°C.

### Example B8

Concentrated H₂SO₄ (1ml) was added to a mixture of 6-[(4-chlorophenyl)hydroxy(1-methyl-1*H*-imidazol-5-yl)methyl]-1-methyl-4-(4-phenyl-2-thiazolyl)-2(1*H*)-quinolinone (see Example B4) in acetonitrile (10ml). The mixture was stirred at 80°C for 45 min and cooled. K₂CO₃ 10% was added. The mixture was extracted with DCM and a small amount of methanol. The organic layer was separated, dried (MgSO₄), filtered and the solvent was evaporated till dryness. The residue (0.81g) was purified by column chromatography over silica gel (eluent: CH₂Cl₂/CH₃OH/NH₄OH 95/5/0.1 and 90/10/0.1; 20-45 µm). The pure fractions were collected and the solvent was evaporated. The residue was crystallized from acetonitrile, 2-propanone and diethyl ether. The precipitate was filtered off and dried, yielding 0.54g (62%) of *N*-[(4-chlorophenyl)[1,2-dihydro-1-methyl-2-oxo-4-(4-phenyl-2-thiazolyl)-6-quinolinyl](1-methyl-1*H*-imidazol-5-yl)methyl]-acetamide, melting point 244°C.

### Example B9

Potassium carbonate (0.0218 mol) and then 2-phenyl-1*H*-imidazole (0.0164 mol) were added to a mixture of intermediate 36 (0.0109 mol) in acetonitrile (60ml). The mixture was stirred and refluxed for 2.5 hours, hydrolysed and extracted with DCM and a small amount of methanol. The organic layer was separated, dried (MgSO₄), filtered and the solvent was evaporated till dryness. The residue (7.3g) was purified by column chromatography over silica gel (eluent: toluene/2-propanol/NH₄OH 87/13/1; 20-45 µm). The pure fractions were collected and the solvent was evaporated. The residue was purified again by column chromatography over C 18 (eluent: H₂O/CH₃OH 20/80). Two pure fractions were collected and their solvents were evaporated, yielding two fractions, firstly 1g F1 (15%) and then 0.5g F2 (8%). F1 was converted into the hydrochloric acid salt (1:1) in 2-propanone. The mixture was crystallized from 2-propanone and DIPE. The precipitate was filtered off and dried, yielding 0.6g (9%) of 6-[(4-chlorophenyl)(2-phenyl-1H-imidazol-1-yl)methyl]-1-methyl-4-(4-phenyl-2-thiazolyl)-2(1*H*)-quinolinone HCl.H₂O, melting point 155°C. F2 was crystallized from CH₃CN, 2-propanone and diethyl ether. The precipitate was filtered off and dried, yielding 0.3g (5%) of 6-[(4-chlorophenyl)(2-phenyl-1H-imidazol-5-yl)methyl]-1-methyl-4-(4-phenyl-2-thiazolyl)- 2(1*H*)-quinolinone, melting point 190°C.

### Example B 10 .

BuLi (1.1ml, 0.0017 mol) was added dropwise at -70°C to a mixture of intermediate 42 (0.0009 mol) in THF (3ml) under N₂ flow. The mixture was stirred at -70°C for 15 minutes. Chlorotriethyl-silane (0.0017 mol) was added dropwise. The mixture was stirred at -70°C for 15 minutes. nBuLi (0.95ml, 0.0015 mol) was added dropwise. The mixture was stirred at -70°C for 15 minutes. A solution of intermediate 41 (0.0009 mol) in THF (4ml) was added at -70°C. The mixture was stirred at -70°C for 15 minutes. H₂O was added. The mixture was extracted with EtOAc. The organic layer was separated, dried (MgSO₄), filtered, and the solvent was evaporated. The residue 0.7g) was purified by column chromatography over silica gel (eluent: CH₂Cl₂/CH₃OH/NH₄OH 95/5/0.5; 15-40µm). The pure fractions were collected and the solvent was evaporated, yielding 0.29g (56%)of 6-[(4-chlorophenyl)hydroxy(1-methyl-1*H*-imidazol-5-yl)methyl]-4-[2-(5-chloro-2-thienyl)ethyl]-1-methyl-2(1*H*)-quinolinone, melting point 128°C.

### Example B11

A mixture of 6-[(4-chlorophenyl)hydroxy(1-methyl-1*H*-imidazol-5-yl)methyl]-4-[2-(5-chloro-2-thienyl)ethyl]-1-methyl- 2(1*H*)-quinolinone), obtained in Example B10 (0.0002 mol) and CH₃COONH₄ (1g) was stirred at 140°C for 30 minutes, then cooled. Water and DCM were added. The mixture was extracted with DCM. The organic layer was separated, dried (MgSO₄), filtered, and the solvent was evaporated. The residue (0.09g) was purified twice by column chromatography over kromasil (eluent: CH₂Cl₂/CH₃OH 100/0 to 90/10; 10µm then toluene/iPrOH 80/20; 5µm). The pure fractions were collected and the solvent was evaporated, yielding 0.025g of 6-[amino(4-chlorophenyl)(1-methyl-1*H*-imidazol-5-yl)methyl]-4-[2-(5-chloro-2-thienyl)ethyl]-1-methyl-2(1*H*)-quinolinone, MS (MH⁺) m/z: 523, 525, 527.

### Example B 12

A mixture of 6-[amino(4-chlorophenyl)(1-methyl-1*H*-imidazol-5-yl)methyl]-4-[2-(5-chloro-2-thienyl)ethyl]-1-methyl-2(1*H*)-quinolinone, obtained in Example B11 (0.0009 mol) and 2,2,2-trichloroethyl carbonochloridic acid ester (0.0047 mol) in THF (5ml) was stirred and refluxed for 48 hours, then cooled and poured out into ice water. The organic layer was extracted with EtOAc, dried (MgSO₄), filtered and the solvent was evaporated. The residue (1.7g) was purified by column chromatography over silica gel (eluent: CH₂Cl₂/CH₃OH/NH₄OH 95/5/0.1; 15-40µm). The pure fractions were collected and the solvent was evaporated, yielding 0.06g (9%) of [(4-chlorophenyl)[4-[2-(5-chloro-2-thienyl)ethyl]-1,2-dihydro-1-methyl-2-oxo-6-quinolinyl](1-methyl-1*H-*imidazol-5-yl)methyl]-carbamic acid, 2,2,2-trichloroethyl ester, MS (MH⁺) m/z: 697, 699, 701, 703, 705, 707.

### Example B13

BuLi (0.0061 mol) was added dropwise at -70°C to a mixture of 3-bromo-pyridine (0.0056 mol) in diethyl ether (50ml) under N₂ flow. The mixture was stirred for 30 minutes. Intermediate 42 (0.0047 mol) was added portionwise. THF (30ml) was added. The mixture was stirred at -70°C for 45 minutes, poured out into water and extracted with EtOAc. The organic layer was separated, dried (MgSO₄) filtered, and the solvent was evaporated. The residue (1.9g) was purified by column chromatography over silica gel (eluent: CH₂Cl₂/CH₃OH/NH₄OH 98/2/0.1; 15-40µm). The pure fractions were collected and the solvent was evaporated, yielding 0.808g (33%) of 6-[(4-chlorophenyl)hydroxy-3-pyridinylmethyl]-4-[2-(5-chloro-2-thienyl)ethyl]-1-methyl-2(1*H*)-quinolinone, melting point 110°C.

### Example B14

a) A mixture of 6-[(4-chlorophenyl)hydroxy-3-pyridinylmethyl]-4-[2-(5-chloro-2-thienyl)ethyl]-1-methyl-2(1*H*)-quinolinone, obtained in Example B13, (0.0009 mol) in thionyl chloride (5ml) was stirred at room temperature for 3 hours. The solvent was evaporated till dryness, yielding 0.55g of 6-[chloro(4-chlorophenyl)-3-pyridinylmethyl]-4-[2-(5-chloro-2-thienyl)ethyl]-1-methyl- 2(1*H*)-quinolinone hydrochloride. This product was used directly in the next reaction step.
b) NH₃/iPrOH (10ml) was added dropwise at 10°C to a mixture of 6-[chloro(4-chlorophenyl)-3-pyridinylmethyl]-4-[2-(5-chloro-2-thienyl)ethyl]-1-methyl-2(1*H*)-quinolinone hydrochloride (0.0009 mol) in THF (5ml). The mixture was brought to room temperature, stirred for 2 hours, poured out into ice water and extracted with EtOAc. The organic layer was separated, dried (MgSO₄) filtered and the solvent was evaporated. The residue (0.56g) was purified by column chromatography over kromasil (eluent: toluene/iPrOH/NEt₃ 92/8/0.3 to CH₂Cl₂/CH₃OH 97/3; 10µm). The pure fractions were collected and the solvent was evaporated, yielding 0.25g (50%) of 6-[amino(4-chlorophenyl)-3-pyridinylmethyl]-4-[2-(5-chloro-2-thienyl)ethyl]-1-methyl-2(1*H*)-quinolinone, melting point 100°C.

The following compounds were prepared analogous to the one of the above examples (the example number analogous to which they were prepared is indicated between square brackets).

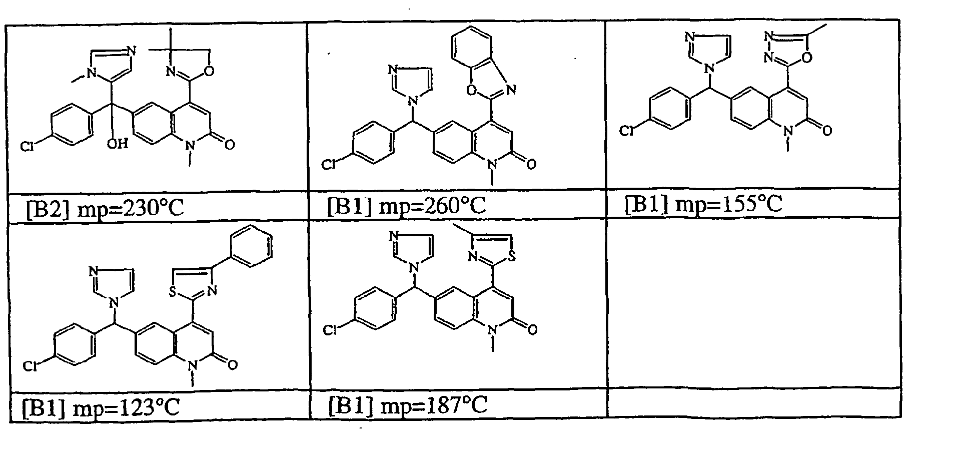

### C. Pharmacological example.

### Example C.1: "In Vitro Assay for Inhibition of Farnesyl Protein Transferase" :

An in vitro assay for inhibition of farnesyl transferase was performed essentially as described in WO 98/40383 , pages 33-34.

### Example C.2: "Ras-Transformed Cell Phenotype Reversion Assay".

The *ras*-transformed cell phenotype reversion assay was performed essentially as described in WO 98/40383 , pages 34-36.

### Example C.3 : "Farnesyl Protein Transferase Inhibitor Secondary Tumor Model".

The farnesyl protein transferase inhibitor secondary tumor model was used as described in WO 98/40383 , page 37.

### D. Composition example : Film-coated tablets

### Preparation of tablet core

A mixture of 100 g of a compound of formula (I), 570 g lactose and 200 g starch is mixed well and thereafter humidified with a solution of 5 g sodium dodecyl sulfate and 10 g polyvinyl-pyrrolidone in about 200 ml of water. The wet powder mixture is sieved, dried and sieved again. Then there are added 100 g microcrystalline cellulose and 15 g hydrogenated vegetable oil. The whole is mixed well and compressed into tablets, giving 10,000 tablets, each comprising 10 mg of a compound of formula (I).

### Coating

To a solution of 10 g methyl cellulose in 75 ml of denaturated ethanol there is added a solution of 5 g of ethyl cellulose in 150 ml of dichloromethane. Then there are added 75 ml of dichloromethane and 2.5 ml 1,2,3-propanetriol. 10 g of polyethylene glycol is molten and dissolved in 75 ml of dichloromethane. The latter solution is added to the former and then there are added 2.5 g of magnesium octadecanoate, 5 g of polyvinyl-pyrrolidone and 30 ml of concentrated colour suspension and the whole is homogenated. The tablet cores are coated with the thus obtained mixture in a coating apparatus.

## Claims

1. A compound of formula (I): 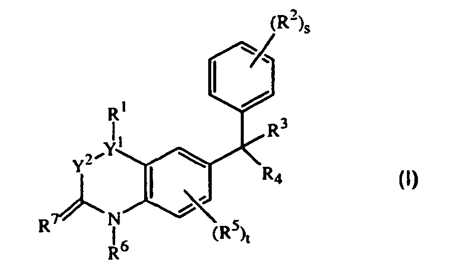 or a pharmaceutically acceptable salt or N-oxide or stereochemically isomeric form thereof, wherein
s is 0 or 1;
t is 0;
>Y¹-Y² - is a trivalent radical of formula
>C=N- (y-1)
or
>C=CR⁹- (y-2)
wherein R⁹ is hydrogen, halo, C₁₋₆alkyl, hydroxycarbonyl or C₁₋₆alkyloxycarbonyl;
R¹ is a group of formula -Z-Het² in which Z is a bond or a -(CH₂)₂- group and Het² is a monocyclic 5- or 6-membered heterocyclic ring containing one, two or three heteroatoms selected from oxygen, sulphur and nitrogen or a bicyclic 9- or 10-membered heterocyclic ring in which a benzene ring is fused to a heterocyclic ring containing one, two or three heteroatoms selected from oxygen, sulphur and nitrogen and optionally substituted by one or two substituents each independently selected from halo, C₁₋₆alkyl or phenyl;
R² is halo, cyano, nitro, -CHO, -CR²⁴=N-OR²⁵ in which R²⁴ is hydrogen and R²⁵ is hydrogen or C₁₋₆alkyl, or two R² substituents ortho to one another on the phenyl ring may independently form together a bivalent radical of formula (a-1);
-O-CH₂-O- (a-1)
R³ is hydrogen or a group of formula (b-1) or (b-3)
-O-R¹⁰ (b-1)
-NR¹¹R¹² (b-3)
wherein R¹⁰ is hydrogen or a group of formula -Alk-OR¹³.
R¹¹ is hydrogen;
R¹² is hydrogen, C₁₋₆alkyl, C₁₋₆alkylcarbonyl, hydroxy or C₁₋₆alkyloxy;
Alk is C₁₋₆alkanediyl and R¹³ is hydrogen;
R⁴ is a group of formula (c-2) or (c-3) of formula 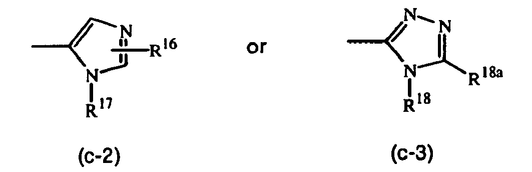 wherein R¹⁶ is hydrogen, halo or C₁₋₆alkyl,
R¹⁷ is hydrogen or C₁₋₆alkyl;
R¹⁸ is hydrogen or C₁₋₆alkyl;
R^{18a} is hydrogen;
R⁶ is hydrogen, -(CR²⁰R²¹)ₚ-C₃₋₁₀cycloalkyl, -C₁₋₆alkylCO₂R²⁴;
-C₁₋₆alkyl CONR²² R²³, -Alk-Ar² or -AlkHet² or C₁₋₆alkyl;
p is 0 to 5;
R²⁰ and R²¹ are independently hydrogen or C₁₋₆ alkyl and are independently defined for each iteration of p in excess of 1;
R²² and R²³ are independently hydrogen, C₁₋₆ alkyl or
-(CR²⁰R²¹)ₚ-C₃₋₁₀cycloalkyl, or together with the adjacent nitrogen atom form a 5- or 6-membered heterocyclic ring optionally containing one, two or three further heteroatoms selected from oxygen, nitrogen or sulphur and optionally substituted by one or two substituents each independently selected from halo, hydroxy, cyano, nitro, C₁₋₆alkyl, haloC₁₋₆alkyl, C₁₋₆alkyloxy, OCF₃, hydroxycarbonyl, C₁₋₆alkyloxycarbonyl, aminocarbonyl, mono- or di-(C₁₋₆alkyl)aminocarbonyl, amino, mono- or di-(C₁₋₆alkyl)amino, C₁₋₆alkylsulfonylamino, oxime, or phenyl;
R²⁴ is hydrogen, C₁₋₆ alkyl, -(CR₂₀R₂₁)p-C₃₋₁₀cycloalkyl or arylC₁₋₆alkyl;
R⁷ is oxygen or sulphur; or R⁶ and R⁷ together form a trivalent radical of formula (x-1), (x-2), (x-3), (x-4) or (x-9):
-CR³⁰=CR³¹-N= (x-1)
-CR³⁰=N-N= (x-2)
-C(=O)-NH-N= (x-3)
-N=N-N= (x-4)
or
-N=N-CR³⁰= (x-9)
wherein each R³⁰, R³¹ and R³² are independently hydrogen, C₁₋₆ alkyl, -OR²⁴, -COOR²⁴, -NR²²R²³, -C₁₋₆ alkylOR²⁴, -C₁₋₆ alkylSR²⁴, R²³R²²NC₁₋₆alkyl-, -CONR²²R²³, C₂₋₆alkenyl, C₂₋₆alkenylAr², C₂₋₆alkenylHet², cyano, amino, thio, C₁₋₆ alkylthio, -O-Ar², -S-Ar² or Ar²;
Ar² is phenyl, naphthyl or phenyl or naphthyl substituted by one to five substituents each independently selected from halo, hydroxy, cyano, nitro, C₁₋₆alkyl, haloC₁₋₆alkyl, - C₁₋₆alkylNR²²R²³, C₁₋₆alkyloxy, OCF₃, hydroxycarbonyl, C₁₋₆alkyloxycarbonyl, aryloxy, -NR²²R²³, C₁₋₆alkylsulfonylamino, oxime or phenyl, or a bivalent substituent of formula -O-CH₂-O- or -O-CH₂-CH₂-O-.

2. A compound according to claim 1 in which:
s is 1, t is 0, >Y¹-Y² is a trivalent radical of formula (y-1) or (y-2), R¹ is a group of formula -Z-Het² in which Z is a bond or a -(CH₂)₂- group and Het² is a monocyclic 5- or 6-membered heterocyclic ring containing one, two or three heteroatoms selected from oxygen, sulphur and nitrogen or a bicyclic 9- or 10-membered heterocyclic ring in which a benzene ring is fused to a heterocyclic ring containing one, two or three heteroatoms selected from oxygen, sulphur and nitrogen and optionally substituted by one or two substituents each independently selected from halo, C₁₋₆alkyl or phenyl, R² is halo or cyano, R³ is hydrogen or a radical of formula (b-1) or (b-3) wherein R¹⁰ is hydrogen or -Alk-OR¹³, R¹¹ is hydrogen, R¹² is hydrogen or C₁₋₆alkylcarbonyl and R¹³ is hydrogen; R⁴ is a radical of formula (c-2) or (c-3) wherein R¹⁶ is hydrogen, R¹⁷ is C₁₋₆alkyl, R¹⁸ is C₁₋₆alkyl and R^{18a} is hydrogen; R⁶ is hydrogen, C₁₋₆alkyl, -CH₂ -C₃₋₁₀cycloalkyl, -C₁₋₆alkylCO₂R²⁴ (R²⁴=H or Et), aminocarbonylC₁₋₆alkyl,-Alk-Ar² or -AlkHet² ; R⁷ is oxygen or sulphur; or R⁶ and R⁷ together form a trivalent radical of formula (x-2), (x-3) or (x-4).

3. A compound according to claim 1 or claim2 in which: s is 1, t is 0, >Y¹-Y² is a trivalent radical of formula (y-1) or (y-2), R¹ is a group of formula -Z-Het² in which Z is a bond or a -(CH₂)₂- group and Het² is a monocyclic 5- or 6-membered heterocyclic ring containing one, two or three heteroatoms selected from oxygen, sulphur and nitrogen, R² is halo or cyano, R³ is hydrogen or a radical of formula (b-1) or (b-3) wherein R⁹ is hydrogen, R¹⁰ is hydrogen, R¹¹ is hydrogen and R¹² is hydrogen or C₁₋₆alkylcarbonyl; R⁴ is a radical of formula (c-2) or (c-3) wherein R¹⁶ is hydrogen, R¹⁷ is C₁₋₆alkyl, R¹⁸ is C₁₋₆alkyl and R^{18a} is hydrogen; R⁶ is hydrogen, C₁₋₆alkyl,
-CH₂-C₃₋₁₀cycloalkyl or -C₁₋₆alkylAr²;
R⁷ is oxygen or sulphur; or R⁶ and R⁷ together form a trivalent radical of formula (x-2) or (x-4).

4. A compound according to any of the preceding claims in which:
s is 1, t is 0, >Y¹-Y² is a trivalent radical of formula (y-1) or (y-2), R¹ is a group of formula -Z-Het² in which Z is a bond or a -(CH₂)₂- group and Het² is selected from thiophene, pyridyl and thiazolyl, optionally substituted by chloro, methyl or phenyl, R² is 4-chloro or 4-cyano, R³ is a radical of formula (b-1) or (b-3) wherein R⁹ is hydrogen, R¹⁰ and R¹¹ are hydrogen and R¹² is hydrogen or C₁₋₆alkylcarbonyl; R⁴ is a radical of formula (c-2) or (c-3) wherein R¹⁶ is hydrogen, R¹⁷ is C₁₋₆alkyl preferably methyl, R¹⁸ is C₁₋₆alkyl preferably methyl and R^{18a} is hydrogen; R⁶ is hydrogen, C₁₋₆alkyl,
-CH₂-C₃₋₁₀cycloalkyl or -C₁₋₆alkylAr²; R⁷ is oxygen or sulphur; or R⁶ and R⁷ together form a trivalent radical of formula (x-4).

5. A compound according to claim 1 selected from:-
6-[(4-chlorophenyl)hydroxy(1-methyl-1*H*-imidazol-5-yl)methyl]-1-methyl-4-(2-thienyl)- 2(1*H*)-quinolinone
6-[(4-chlorophenyl)hydroxy(1-methyl-1*H*-imidazol-5-yl)methyl]-4-(3-pyridinyl)-2(1*H*)-quinolinone,
6-[(4-chlorophenyl)hydroxy(1-methyl-1*H*-imidazol-5-yl)methyl]-1-methyl-4-(4-methyl-2-thiazolyl)- 2(1*H*)-quinolinone
*N*-[(4-chlorophenyl)[1,2-dihydro-1-methyl-2-oxo-4-(4-phenyl-2-thiazolyl)-6-quinolinyl](1-methyl-1*H*-imidazol-5-yl)methyl]- acetamide and
6-[amino(4-chlorophenyl)(1-methyl-1*H*-imidazol-5-yl)methyl]-4-[2-(5-chloro-2-thienyl)ethyl]-1-methyl-2(1*H*)-quinolinone
and their pharmaceutically acceptable salts.

6. A process for the preparation of a compound as claimed in claim 1 which comprises:-
a) cyclising a compound of formula (II): 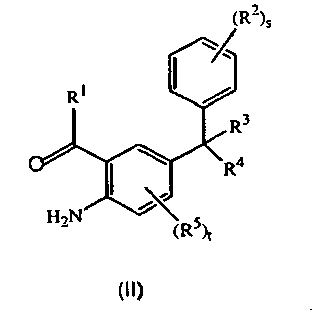 with a reagent serving to form a compound of formula (I) in which Z is a bond, R⁶ is hydrogen and R⁷ is oxygen;
b) reacting a compound of formula (III): 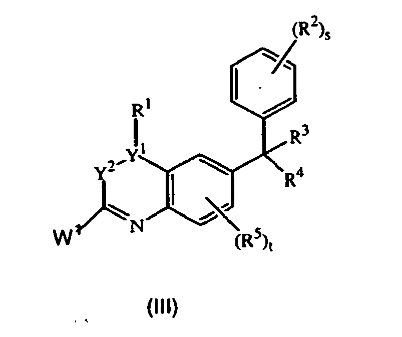 in which W¹ represents a replaceable or reactive group, with a reagent serving either to react with or replace the W¹ group in compound (III) to form a compound of formula (I) in which R⁶ is hydrogen and R⁷ is an oxygen or sulphur group or to react with the W¹ group and the adjacent nitrogen atom to form directly or indirectly a compound of formula (I) in which R⁶ and R⁷ together form a trivalent radical selected from formulae (x-1) to (x-10); or
c) reacting a compound of formula (IV): 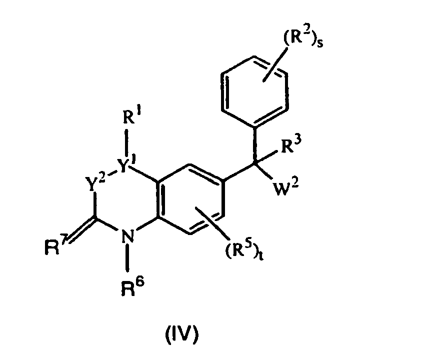 in which W² is a replaceable group, with an imidazole reagent serving to replace the group W² with an R⁴ group of formula (c-1); or
d) reacting a compound of formula (V): 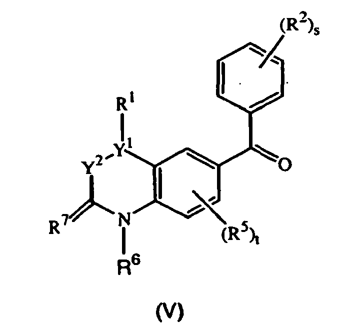 with an imidazole reagent to form a compound of formula (I) in which R⁴ is a group of formula (c-2)), or with a 3-mercapto-4-C₁₋₆alkyl-1,2,4-triazole reagent to form the corresponding 3-mercapto-4-C₁₋₆alkyl-1,2,4-triazole derivative, which is optionally methylated to form the corresponding 3-methylmercapto derivative, and subsequently removing the 3-mercapto or 3-methylmercapto group to form a compound of formula (I) in which R⁴ is a group of formula (c-3) in which R¹⁸ is a C₁₋₆alkyl group; or with a 3-bromopyridyl reagent to form a compound of formula (I) wherein R⁴ is a group of formula (c-4); or
e) reacting a compound of formula (VI): 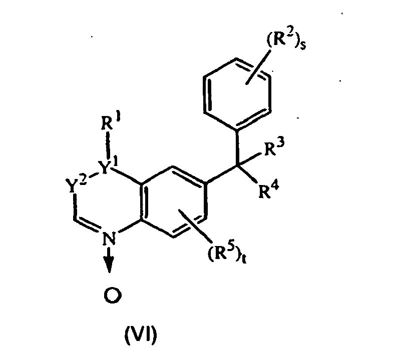 with a reagent serving to convert the said compound (VI) to a compound of formula (I) in which R^{**6**} is hydrogen and R⁷ is oxygen; and optionally effecting one or more of the following conversions in any desired order:-
(i) converting a compound of formula (I) into a different compound of formula (I);
(ii) converting a compound of formula (I) in to a pharmaceutically acceptable salt or N-oxide thereof;
(iii) converting a pharmaceutically acceptable salt or N-oxide of a compound of formula (I) into the parent compound of formula (I);
(iv) preparing a stereochemical isomeric form of a compound of formula (I) or a pharmaceutically acceptable salt or N-oxide thereof.

7. A compound according to any of claims 1 to 5 for use as a medicine.

8. A compound according to claim 7 for use in inhibiting tumor growth.

9. Use of a compound according to claim 1 in the manufacture of a medicament for inhibiting tumor growth by administering an effective amount of the medicament to a subject, in need of such treatment.

## Revendications

1. Composé de formule (I) : 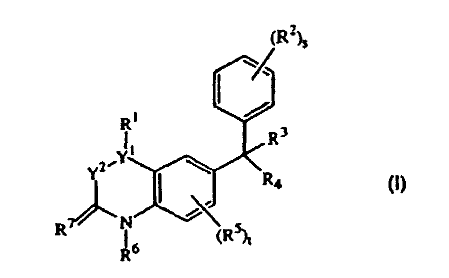 ou un sel pharmaceutiquement acceptable ou un N-oxyde ou une forme stéréochimiquement isomère de celui-ci,
dans laquelle
s vaut 0 ou 1;
t vaut 0 ;
>Y¹-Y²- est un radical trivalent de formule
>C=N- (y-1)
ou
>C=CR⁹- (y-2)
dans laquelle R⁹ est hydrogène, halogéno, alkyl(C₁₋₆), hydroxycarbonyle ou alkyl(C₁₋₆)oxycarbonyle ;
R¹ est un groupement de formule -Z-Het²
dans laquelle Z est une liaison ou un groupement -(CH₂)₂- et Het² est un cycle hétérocyclique de 5 ou 6 chaînons monocyclique contenant un, deux ou trois hétéroatomes choisis parmi l'oxygène, le soufre et l'azote, ou un cycle hétérocyclique de 9 ou 10 chaînons bicyclique dans lequel un cycle benzénique est condensé sur un cycle hétérocyclique contenant un, deux ou trois hétéroatomes choisis parmi l'oxygène, le soufre et l'azote, et éventuellement substitué par un ou deux substituants choisis chacun indépendamment parmi halogéno, alkyl(C₁₋₆) ou phényle ;
R² est halogéno, cyano, nitro, -CHO, -CR²⁴=N-OR²⁵ où R²⁴ est hydrogène et R²⁵ est hydrogène ou alkyl(C₁₋₆), ou deux substituants R² en ortho l'un par rapport à l'autre sur le cycle phényle peuvent indépendamment former ensemble un radical bivalent de formule (a-1) ;
-O-CH₂-O- (a-1)
R³ est hydrogène ou un groupement de formule (b-1) ou (b-3)
-O-R¹⁰ (b-1)
-NR¹¹R¹² (b-3)
dans laquelle R¹⁰ est hydrogène ou un groupement de formule -Alk-OR¹³,
R¹¹ est hydrogène;
R¹² est hydrogène, alkyl(C₁₋₆), alkyl(C₁₋₆)carbonyle, hydroxy ou alkyl(C₁₋₆)oxy ;
Alk est alcane(C₁₋₆)diyle et R¹³ est hydrogène ;
R⁴ est un groupement de formule (c-2) ou (c-3) de formule 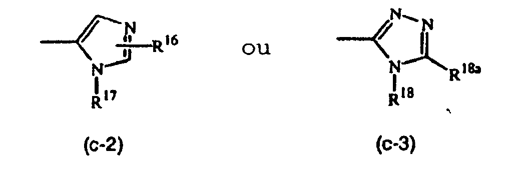 dans laquelle R¹⁶ est hydrogène, halogéno ou alkyl(C₁₋₆),
R¹⁷ est hydrogène ou alkyl(C₁₋₆),
R¹⁸ est hydrogène ou alkyl(C₁₋₆),
R^{18a} est hydrogène ;
R⁶ est hydrogène, -(CR²⁰R²¹)ₚ-cycloalkyl (C₃₋₁₀), -alkyl(C₁₋₆) CO₂R²⁴; -alkyl(C₁₋₆) -CONR²²R²³, -Alk-Ar² ou AlkHet² ou alkyl(C₁₋₆) ;
p va de 0 à 5 ;
R²⁰ et R²¹ sont indépendamment hydrogène ou alkyl (C₁₋₆) et sont indépendamment définis pour chaque itération de p au-dessus de 1 ;
R²² et R²³ sont indépendamment hydrogène, alkyl (C₁₋₆) ou - (CR²⁰R²¹)ₚ-cycloalkyl (C₃₋₁₀), ou, conjointement avec l'atome d'azote adjacent, forment un cycle hétérocyclique de 5 ou 6 chaînons contenant éventuellement un, deux ou trois hétéroatomes supplémentaires choisis parmi l'oxygène, l'azote ou le soufre et éventuellement substitué par un ou deux substituants choisis chacun indépendamment parmi halogéno, hydroxy, cyano, nitro, alkyl(C₁₋₆), halogénoalkyl(C₁₋₆), alkyl (C₁₋₆) oxy, OCF₃, hydroxycarbonyle, alkyl(C₁₋₆)oxycarbonyle, aminocarbonyle, mono- ou di-(alkyl(C₁₋₆))aminocarbonyle, amino, mono- ou di (alkyl (C₁₋₆)) amino, alkyl (C₁₋₆) sulfonylamino, oxime, ou phényle ;
R²⁴ est hydrogène, alkyl (C₁₋₆), -(CR²⁰R²¹)ₚ-cycloalkyl (C₃₋₁₀) ou arylalkyl(C₁₋₆);
R⁷ est oxygène ou soufre ; ou R⁶ et R⁷ forment ensemble un radical trivalent de formule (x-1), (x-2), (x-3), (x-4) ou (x-9) :
-CR³⁰=CR³¹-N= (x-1)
-CR³⁰=N-N= (x-2)
-C(=O)-NH-N= (x-3)
-N=N-N= (x-4)
ou
-N=N-CR³⁰= (x-9)
dans laquelle chaque R³⁰, R³¹ et R³² sont indépendamment hydrogène, alkyl(C₁₋₆), -OR²⁴, -COOR²⁴, -NR²²R²³, -alkyl(C₁₋₆) OR²⁴, -alkyl (C₁₋₆) SR²⁴, R²³R²²Nalkyl(C₁₋₆)-, -CONR²²R²³, alcényl(C₂₋₆), alcényl(C₂₋₆)Ar², alcényl (C₂₋₆) Het², cyano, amino, thio, alkyl(C₁₋₆)thio, -O-Ar², -S-Ar² ou Ar² ;
Ar² est phényle, naphtyle ou phényle ou naphtyle substitué par de un à cinq substituants choisis chacun indépendamment parmi halogéno, hydroxy, cyano, nitro, alkyl (C₁₋₆), halogénoalkyl (C₁₋₆), -alkyl(C₁₋₆)NR²²R²³, alkyl(C₁₋₆)oxy, OCF₃, hydroxycarbonyle, alkyl(C₁₋₆)-oxycarbonyle, aryloxy, -NR²²R²³, alkyl(C₁₋₆)sulfonylamino, oxime ou phényle, ou un substituant bivalent de formule -O-CH₂-O- ou -O-CH₂-CH₂-O-.

2. Composé selon la revendication 1, dans lequel:
s vaut 1, t vaut 0, >Y¹-Y²- est un radical trivalent de formule (y-1) ou (y-2), R¹ est un groupement de formule -Z-Het² dans laquelle Z est une liaison ou un groupement -(CH₂)₂- et Het² est un cycle hétérocyclique de 5 ou 6 chaînons monocyclique contenant un, deux ou trois hétéroatomes choisis parmi l'oxygène, le soufre et l'azote, ou un cycle hétérocyclique de 9 ou 10 chaînons bicyclique dans lequel un cycle benzénique est condensé sur un cycle hétérocyclique contenant un, deux ou trois hétéroatomes choisis parmi l'oxygène, le soufre et l'azote, et éventuellement substitué par un ou deux substituants choisis chacun indépendamment parmi halogéno, alkyl(C₁₋₆) ou phényle,
R² est halogéno ou cyano, R³ est hydrogène ou un radical de formule (b-1) ou (b-3) dans laquelle R¹⁰ est hydrogène ou -Alk-OR¹³, R¹¹ est hydrogène, R¹² est hydrogène ou alkyl (C₁₋₆) carbonyle, et R¹³ est hydrogène ; R⁴ est un radical de formule (c-2) ou (c-3) dans laquelle R¹⁶ est hydrogène, R¹⁷ est alkyl(C₁₋₆), R¹⁸ est alkyl(C₁₋₆) et R^{18a} est hydrogène; R⁶ est hydrogène, alkyl (C₁₋₆), -CH₂-cycloalkyl (C₃₋₁₀), -alkyl (C₁₋₆)CO₂R²⁴ (R²⁴= H ou Et), aminocarbonylalkyl(C₁₋₆), -Alk-Ar² ou AlkHet² ; R⁷ est oxygène ou soufre ; ou R⁶ et R⁷ forment ensemble un radical trivalent de formule (x-2), (x-3) ou (x-4).

3. Composé selon la revendication 1 ou la revendication 2, dans lequel :
s vaut 1, t vaut 0, >Y¹-Y²- est un radical trivalent de formule (y-1) ou (y-2), R¹ est un groupement de formule -Z-Het² dans laquelle Z est une liaison ou un groupement - (CH₂)₂- et Het² est un cycle hétérocyclique de 5 ou 6 chaînons monocyclique contenant un, deux ou trois hétéroatomes choisis parmi l'oxygène, le soufre et l'azote, R² est halogéno ou cyano, R³ est hydrogène ou un radical de formule (b-1) ou (b-3) dans laquelle R⁹ est hydrogène, R¹⁰ est hydrogène, R¹¹ est hydrogène et R¹² est hydrogène ou alkyl(C₁₋₆)carbonyle; R⁴ est un radical de formule (c-2) ou (c-3) dans laquelle R¹⁶ est hydrogène, R¹⁷ est alkyl (C₁₋₆), R¹⁸ est alkyl (C₁₋₆) et R^{18a} est hydrogène ; R⁶ est hydrogène, alkyl (C₁₋₆), -CH₂₋cycloalkyl (C₃₋₁₀) ou -alkyl(C₁₋₆)Ar² ; R⁷ est oxygène ou soufre ; ou R⁶ et R⁷ forment ensemble un radical trivalent de formule (x-2) ou (x-4).

4. Composé selon l'une quelconque des revendications précédentes, dans lequel :
s vaut 1, t vaut 0, >Y¹-Y²- est un radical trivalent de formule (y-1) ou (y-2), R¹ est un groupement de formule -Z-Het² dans laquelle Z est une liaison ou un groupement -(CH₂)₂- et Het² est choisi parmi thiophène, pyridyle et thiazolyle, éventuellement substitué par chloro, méthyle ou phényle, R² est 4-chloro ou 4-cyano, R³ est un radical de formule (b-1) où (b-3) dans laquelle R⁹ est hydrogène, R¹⁰ et R¹¹ sont hydrogène et R¹² est hydrogène ou alkyl(C₁₋₆)carbonyle ; R⁴ est un radical de formule (c-2) ou (c-3) dans laquelle R¹⁶ est hydrogène, R¹⁷ est alkyl (C₁₋₆) préférablement méthyle, R¹⁸ est alkyl(C₁₋₆), préférablement méthyle, et R^{18a} est hydrogène ; R⁶ est hydrogène, alkyl (C₁₋₆), -CH₂₋cycloalkyl(C₃₋₁₀) ou -alkyl(C₁₋₆)Ar² ; R⁷ est oxygène ou soufre ; ou R⁶ et R⁷ forment ensemble un radical trivalent de formule (x-4).

5. Composé selon la revendication 1, choisi parmi :
la 6-[(4-chlorophényl)hydroxy(1-méthyl-1*H*-imidazol-5-yl)méthyl]-1-méthyl-4-(2-thiényl)-2(1*H*)quinolinone,
la 6-[(4-chlorophényl)hydroxy(1-méthyl-1*H*-imidazol-5-yl)méthyl]-4-(3-pyridinyl)-2(1*H*)quinolinone,
la 6-[(4-chlorophényl)hydroxy(1-méthyl-1*H*-imidazol-5-yl)méthyl]-1-méthyl-4-(4-méthyl-2-thiazolyl)-2(1*H*)-quinolinone,
le *N*-[(4-chlorophényl)[1,2-dihydro-1-méthyl-2-oxo-4-(4-phényl-2-thiazolyl)-6-quinoléinyl](1-méthyl-1*H-*imidazol-5-yl)méthyl]acétamide, et
la 6-[amino(4-chlorophényl)(1-méthyl-1*H*-imidazol-5-yl)méthyl]-4-[2-(5-chloro-2-thiényl)éthyl]-1-méthyl-2(1*H*)quinolinone,
et leurs sels pharmaceutiquement acceptables.

6. Procédé de préparation d'un composé selon la revendication 1, comprenant :
a) la cyclisation d'un composé de formule (II) : 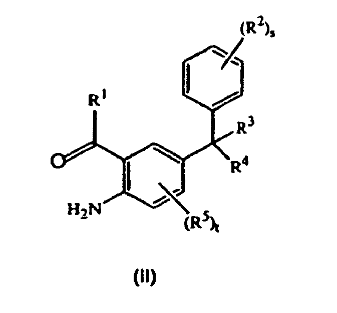 avec un réactif servant à former un composé de formule (I) dans laquelle Z est une liaison, R⁶ est hydrogène et R⁷ est oxygène ;
b) la réaction d'un composé de formule (III) : 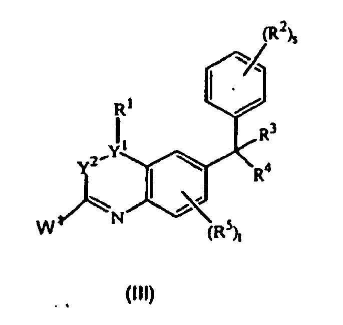 dans laquelle W¹ représente un groupement remplaçable ou réactif, avec un réactif servant soit à réagir avec ou remplacer le groupement W¹ dans le composé (III) pour former un composé de formule (I) dans laquelle R⁶ est hydrogène et R⁷ est un groupement oxygène ou soufre, soit à réagir avec le groupement W¹ et l'atome d'azote adjacent pour former directement ou indirectement un composé de formule (I) dans laquelle R⁶ et R⁷ forment ensemble un radical trivalent choisi parmi les formules (x-1) à (x-10) ; ou
c) la réaction d'un composé de formule (IV) : 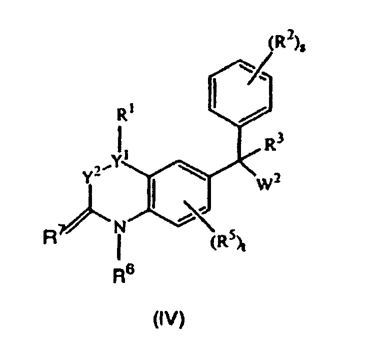 dans laquelle W² est un groupement remplaçable, avec un réactif imidazolique servant à remplacer le groupement W² par un groupement R⁴ de formule (c-1) ; ou
d) la réaction d'un composé de formule (V) : 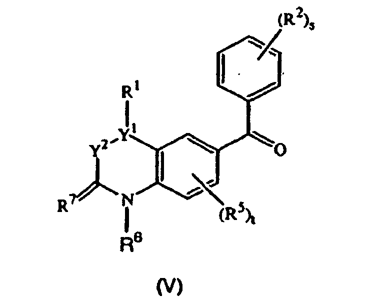 avec un réactif imidazolique pour former un composé de formule (I) dans laquelle R⁴ est un groupement de formule (c-2), ou avec un réactif au 3-mercapto-4-alkyl(C₁₋₆)-1,2,4-triazole pour former le dérivé de 3-mercapto-4-alkyl(C₁₋₆)-1,2,4-triazole correspondant, qui est éventuellement méthylé pour former le dérivé de 3-méthylmercapto correspondant, et ensuite l'élimination du groupement 3-mercapto ou 3-méthylmercapto pour former un composé de formule (I) dans laquelle R⁴ est un groupement de formule (c-3) dans laquelle R¹⁸ est un groupement alkyl(C₁₋₆); ou avec un réactif au 3-bromopyridyle pour former un composé de formule (I) dans laquelle R⁴ est un groupement de formule (c-4) ; ou
e) la réaction d'un composé de formule (VI) : 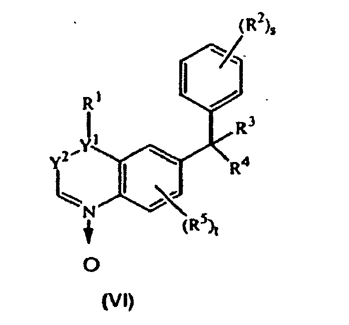 avec un réactif servant à convertir ledit composé (VI) en un composé de formule (I) dans laquelle R⁶ est hydrogène et R⁷ est oxygène ; et éventuellement effectuer une ou plusieurs parmi les conversions suivantes selon un ordre désiré quelconque :
(i) la conversion d'un composé de formule (I) en un composé de formule (I) différent ;
(ii) la conversion d'un composé de formule (I) en un sel pharmaceutiquement acceptable ou un N-oxyde de celui-ci ;
(iii) la conversion d'un sel pharmaceutiquement acceptable ou d'un N-oxyde d'un composé de formule (I) en composé de formule (I) parent;
(iv) la préparation d'une forme d'isomère stéréochimique d'un composé de formule (I) ou d'un sel pharmaceutiquement acceptable ou d'un N-oxyde de celui-ci.

7. Composé selon l'une quelconque des revendications 1 à 5, pour une utilisation comme médicament.

8. Composé selon la revendication 7, pour une utilisation dans l'inhibition de la croissance de tumeurs.

9. Utilisation d'un composé selon la revendication 1 dans la fabrication d'un médicament destiné à l'inhibition de la croissance de tumeurs, par l'administration d'une quantité efficace du médicament à un sujet ayant besoin d'un tel traitement.

## Patentansprüche

1. Verbindungen der Formel (I): 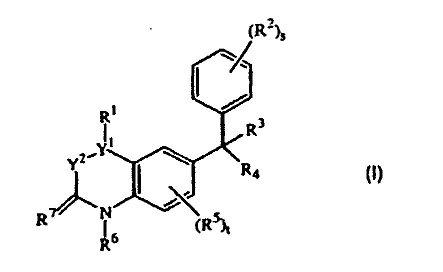 und deren pharmazeutisch unbedenkliche Salze und N-Oxide und stereochemisch isomere Formen, wobei
s für 0 oder 1 steht;
t für 0 steht;
>Y¹-Y²- für einen dreiwertigen Rest der Formel
>C=N- (y-1)
oder
>C=CR⁹- (y-2)
steht,
wobei R⁹ für Wasserstoff, Halogen, C₁₋₆-Alkyl, Hydroxycarbonyl oder C₁₋₆-Alkyloxycarbonyl steht;
R¹ für eine Gruppe der Formel -Z-Het² steht, in welcher Z für eine Bindung oder eine -(CH₂)₂₋Gruppe steht und Het² für einen moncyclischen, 5- oder 6gliedrigen heterocyclischen Ring mit einem, zwei oder drei Heteroatomen ausgewählt aus Sauerstoff, Schwefel und Stickstoff oder einen bicyclischen, 9- oder 10gliedrigen heterocyclischen Ring, in welchem ein Benzolring mit einem heterocyclischen Ring mit einem, zwei oder drei Heteroatomen ausgewählt aus Sauerstoff, Schwefel und Stickstoff und gegebenenfalls substituiert durch einen oder zwei Substituenten jeweils unabhängig voneinander ausgewählt aus Halogen, C₁₋₆-Alkyl oder Phenyl kondensiert ist, steht;
R² für Halogen, Cyano, Nitro, -CHO, -CR²⁴=N-OR²⁵, in welcher R²⁴ für Wasserstoff und R²⁵ für Wasserstoff oder C₁₋₆-Alkyl steht, steht, oder zwei R²-Substituenten ortho zueinander am Phenylring unabhängig zusammen einen zweiwertigen Rest der Formel (a-1)
-O-CH₂-O- (a-1)
bilden können;
R³ für Wasserstoff oder eine Gruppe der Formel (b-1) oder (b-3)
-O-R¹⁰ (b-1)
-NR¹¹R¹² (b-3)
steht,
wobei R¹⁰ für Wasserstoff oder eine Gruppe der Formel -Alk-OR¹³ steht;
R¹¹ für Wasserstoff steht;
R¹² für Wasserstoff, C₁₋₆-Alkyl, C₁₋₆-Alkylcarbonyl, Hydroxy oder C₁₋₆-Alkyloxy steht;
Alk für C₁₋₆-Alkandiyl steht und R¹³ für Wasserstoff steht;
R⁴ für eine Gruppe der Formel (c-1) oder (c-3) 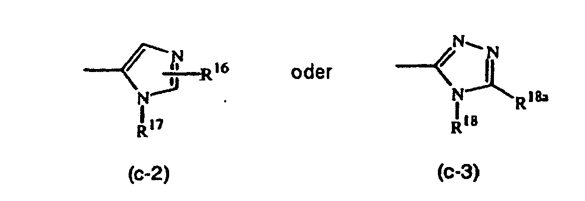 steht, wobei R¹⁶ für Wasserstoff, Halogen oder C₁₋₆-Alkyl steht;
R¹⁷ für Wasserstoff oder C₁₋₆-Alkyl steht;
R¹⁸ für Wasserstoff oder C₁₋₆-Alkyl steht;
R^{18a} für Wasserstoff steht;
R⁶ für Wasserstoff, -(CR²⁰R²¹)ₚ-C₃₋₁₀-Cycloalkyl, -C₁₋₆-Alkyl-CO₂R²⁴, -C₁₋₆-Alkyl-CONR²²R²³, -Alk-Ar² oder -Alk-Het² oder C₁₋₆-Alkyl steht;
p für 0 bis 5 steht;
R²⁰ und R²¹ unabhängig voneinander für Wasserstoff oder C₁₋₆-Alkyl stehen und für jede Iteration von p größer als 1 unabhängig voneinander definiert sind;
R²² und R²³ unabhängig voneinander für Wasserstoff, C₁₋₆-Alkyl oder -(CR²⁰R²¹)ₚ-C₃₋₁₀₋Cycloalkyl stehen oder zusammen mit dem benachbarten Stickstoffatom einen 5- oder 6gliedrigen heterocyclischen Ring bilden, der gegebenenfalls ein, zwei oder drei weitere Heteroatome ausgewählt aus Sauerstoff, Stickstoff und Schwefel enthält und gegebenenfalls durch einen oder zwei Substituenten jeweils unabhängig voneinander ausgewählt aus Halogen, Hydroxy, Cyano, Nitro, C₁₋₆-Alkyl, Halogen-C₁₋₆₋Alkyl, C₁₋₆-Alkyloxy, OCF₃, Hydroxycarbonyl, C₁₋₆₋Alkyloxycarbonyl, Aminocarbonyl, Mono- oder Di-(C₁₋₆-Alkyl)aminocarbonyl, Amino, Mono- oder Di-(C₁₋₆-Alkyl)amino, C₁₋₆-Alkylsulfonylamino, Oxim und Phenyl substituiert ist;
R²⁴ für Wasserstoff, C₁₋₆-Alkyl, -(CR²⁰R²¹)ₚ-C₃₋₁₀₋Cycloalkyl oder Aryl-C₁₋₆-Alkyl steht;
R⁷ für Sauerstoff oder Schwefel steht; oder R⁶ und R⁷ zusammen einen dreiwertigen Rest der Formel (x-1), (x-2), (x-3), (x-4) oder (x-9):
-CR³⁰ =CR³¹ -N= (x-1)
-CR³⁰ =N-N= (x-2)
-C(=O)-NH-N= (x-3)
-N=N-N= (x-4)
oder -N=N-CR³⁰ = (x-9)
bilden, wobei R³⁰, R³¹ und R³² jeweils unabhängig voneinander für Wasserstoff, C₁₋₆-Alkyl, OR²⁴, -COOR²⁴, NR²²R²³, -C₁₋₆-Alkyl-OR²⁴, -C₁₋₆-Alkyl-SR²⁴, R²³R²²N-C₁₋₆-Alkyl- , -CONR²²R²³ , C₂₋₆-Alkenyl, C₂₋₆₋Alkenyl-Ar² C₂₋₆-Alkenyl-Het², Cyano, Amino, Thio, C₁₋₆-Alkylthio, -O-Ar², -S-Ar² oder Ar² stehen;
Ar² für Phenyl, Naphthyl oder durch einen bis fünf Substituenten jeweils unabhängig voneinander ausgewählt aus Halogen, Hydroxy, Cyano, Nitro, C₁₋₆-Alkyl, Halogen-C₁₋₆-alkyl, -C₁₋₆-Alkyl-NR²²R²³, C₁₋₆-Alkyloxy, OCF₃, Hydroxycarbonyl, C₁₋₆₋Alkyloxycarbonyl, Aryloxy, -NR²²R²³, C₁₋₆-Alkyl- sulfonylamino, Oxim und Phenyl oder einen zweiwertigen Substituenten der Formel -O-CH₂-O oder -O-CH₂-CH₂-O substituiertes Phenyl oder Naphthyl steht.

2. Verbindungen nach Anspruch 1, in denen:
s für 1 steht, t für 0 steht, >Y¹-Y² für einen dreiwertigen Rest der Formel (y-1) oder (y-2) steht, R¹ für eine Gruppe der Formel -Z-Het² steht, in welcher Z für eine Bindung oder eine-(CH₂)₂₋Gruppe steht und Het² für einen moncyclischen 5-oder 6gliedrigen heterocyclischen Ring mit einem, zwei oder drei Heteroatomen ausgewählt aus Sauerstoff, Schwefel und Stickstoff oder einen bicyclischen 9- oder 10gliedrigen heterocyclischen Ring, in welchem ein Benzolring an einen heterocyclischen Ring mit einem, zwei oder drei Heteroatomen ausgewählt aus Sauerstoff, Schwefel und Stickstoff und gegebenenfalls substituiert durch einen oder zwei Substituenten jeweils unabhängig voneinander ausgewählt aus Halogen, C₁₋₆-Alkyl oder Phenyl kondensiert ist,
R² für Halogen oder Cyano steht, R³ für Wasserstoff oder einen Rest der Formel (b-1) oder (b-3) steht, wobei R¹⁰ für Wasserstoff oder -Alk-OR¹³ steht, R¹¹ für Wasserstoff steht, R¹² für Wasserstoff oder C₁₋₆-Alkylcarbonyl steht und R¹³ für Wasserstoff steht; R⁴ für einen Rest der Formel (c-2) oder (c-3) steht, wobei R¹⁶ für Wasserstoff steht, R¹⁷ für C₁₋₆-Alkyl steht, R¹⁸ für C₁₋₆-Alkyl steht und R^{18a} für Wasserstoff steht; R⁶ für Wasserstoff, C₁₋₆₋Alkyl, -CH₂-C₃₋₁₀-Cycloalkyl, -C₁₋₆-Alkyl-CO₂R²⁴(R²⁴=H oder Et), Aminocarbonyl-C₁₋₆-alkyl, -Alk-Ar² oder -AlkHet² steht; R⁷ für Sauerstoff oder Schwefel steht; oder R⁶ und R⁷ zusammen einen dreiwertigen Rest der Formel (x-2), ,(x-3) oder (x-4) bilden.

3. Verbindungen nach Anspruch 1 oder 2, in denen:
s für 1 steht, t für 0 steht, >Y¹-Y² für einen dreiwertigen Rest der Formel (y-1) oder (y-2) steht, R¹ für eine Gruppe der Formel -Z-Het² steht, in welcher Z für eine Bindung oder eine - (CH₂)₂₋Gruppe steht und Het² für einen moncyclischen 5-oder 6gliedrigen heterocyclischen Ring mit einem, zwei oder drei Heteroatomen ausgewählt aus Sauerstoff, Schwefel und Stickstoff steht, R² für Halogen oder Cyano steht, R³ für Wasserstoff oder einen Rest der Formel (b-1) oder (b-3) steht, wobei R⁹ für Wasserstoff steht, R¹⁰ für Wasserstoff steht, R¹¹ für Wasserstoff steht und R¹² für Wasserstoff oder C₁₋₆-Alkylcarbonyl steht; R⁴ für einen Rest der Formel (c-2) oder (c-3) steht, wobei R¹⁶ für Wasserstoff steht, R¹⁷ für C₁₋₆-Alkyl steht, R¹⁸ für C₁₋₆-Alkyl steht und R^{18a} für Wasserstoff steht; R⁶ für Wasserstoff, C₁₋₆-Alkyl, -CH₂-C₃₋₁₀-Cycloalkyl oder -C₁₋₆-Alkyl-Ar² steht;
R⁷ für Sauerstoff oder Schwefel steht; oder R⁶ und R⁷ zusammen einen dreiwertigen Rest der Formel (x-2) oder (x-4) bilden.

4. Verbindungen nach einem der vorhergehenden Ansprüche, in denen:
s für 1 steht, t für 0 steht, >Y¹-Y² für einen dreiwertigen Rest der Formel (y-1) oder (y-2) steht, R¹ für eine Gruppe der Formel -Z-Het² steht, in welcher Z für eine Bindung oder eine -(CH₂)₂₋Gruppe steht und Het² ausgewählt ist aus Thiophen, Pyridyl und Thiazolyl, gegebenenfalls substituiert durch Chlor, Methyl oder Phenyl, R² für 4-Chlor oder 4-Cyano steht, R³ für einen Rest der Formel (b-1) oder (b-3) steht, wobei R⁹ für Wasserstoff. steht, R¹⁰ und R¹¹ für Wasserstoff stehen und R¹² für Wasserstoff oder C₁₋₆-Alkylcarbonyl steht; R⁴ für einen Rest der Formel (c-2) oder (c-3) steht, wobei R¹⁶ für Wasserstoff steht, R¹⁷ für C₁₋₆-Alkyl, vorzugsweise Methyl, steht, R¹⁸ für C₁₋₆-Alkyl, vorzugsweise Methyl, steht, und R^{18a} für Wasserstoff steht; R⁶ für Wasserstoff, C₁₋₆-Alkyl, -CH₂₋C₃₋₁₀-Cycloalkyl oder -C₁₋₆-Alkyl-Ar² steht; R⁷ für Sauerstoff oder Schwefel steht; oder R⁶ und R⁷ zusammen einen dreiwertigen Rest der Formel (x-4) bilden.

5. Verbindungen nach Anspruch 1, ausgewählt aus:
6-[(4-Chlorphenyl)hydroxy(1-methyl-1*H*-imidazol-5-yl)methyl]-1-methyl-4-(2-thienyl)-2(1*H*)-chinolinon,
6-[(4-Chlorphenyl)hydroxy(1-methyl-1*H*-imidazol-5-yl)methyl]-4-(3-pyridinyl)-2(1*H*)-chinolinon, 6-[(4-Chlorphenyl)hydroxy(1-methyl-1*H*-imidazol-5-yl)methyl]-1-methyl-4-(4-methyl-2-thiazolyl)-2(1*H*)-chinolinon,
N-[(4-Chlorphenyl)[1,2-dihydro-1-methyl-2-oxo-4-(4-phenyl-2-thiazolyl)-6-chinolinyl](1-methyl-1*H-*imidazol-5-yl)methyl]acetamid und
6-[Amino(4-chlorphenyl)(1-methyl-1*H*-imidazol-5-yl) methyl]-4-[2-(5-chlor-2-thienyl)ethyl]-1-methyl-2(1*H*)-chinolinon
und deren pharmazeutisch unbedenklichen Salzen.

6. Verfahren zur Herstellung einer Verbindung nach Anspruch 1, bei dem man:
a) eine Verbindung der Formel (II): 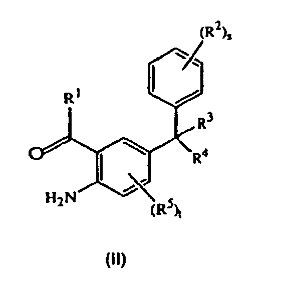 mit einem Reagens cyclisiert, das dazu dient, eine Verbindung der Formel (I) zu bilden, in welcher Z für eine Bindung steht, R⁶ für Wasserstoff steht und R⁷ für Sauerstoff steht;
b) eine Verbindung der Formel (III): 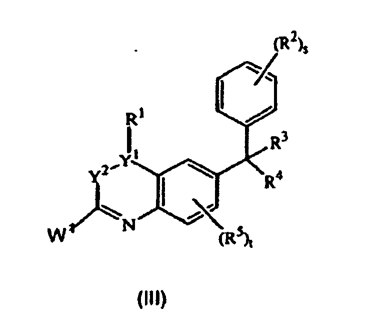 in welcher w¹ für eine Abgangsgruppe bzw. eine reaktive Gruppe steht, mit einem Reagens umsetzt, das dazu dient, entweder mit der W¹⁻Gruppe in der Verbindung (III) zu reagieren oder die W¹-Gruppe in der Verbindung (III) zu ersetzen, unter Bildung einer Verbindung der Formel (I), in welcher R⁶ für Wasserstoff steht und R⁷ für eine Sauerstoff- oder Schwefelgruppe steht, oder mit der W¹-Gruppe und dem benachbarten Stickstoffatom zu reagieren, unter direkter oder indirekter Bildung einer Verbindung der Formel (I), in welcher R⁶ und R⁷ zusammen einen dreiwertigen Rest ausgewählt aus den Formeln ((x-1) bis (x-10) bilden; oder
c) eine Verbindung der Formel (IV): 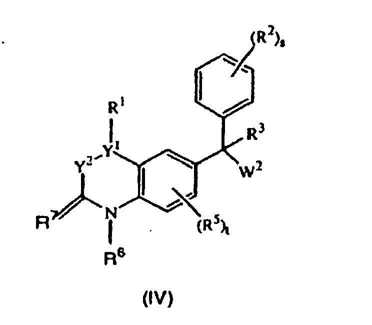 in welcher W² für eine Abgangsgruppe steht, mit einem Imidazolreagens umsetzt, das dazu dient, die Gruppe W² durch eine R⁴-Gruppe der Formel (c-1) zu ersetzen; oder
d) eine Verbindung der Formel (V): 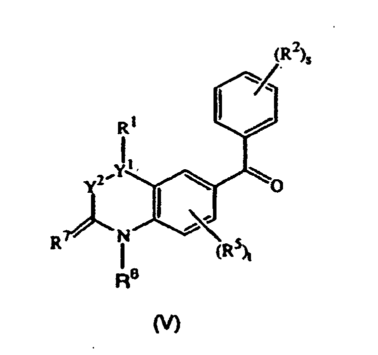 mit einem Imidazolreagens umsetzt, unter Bildung einer Verbindung der Formel (I), in welcher R⁴ für eine Gruppe der Formel (c-2) steht, oder mit einem 3-Mercapto-4-C₁₋₆alkyl-1,2,4-triazolreagens umsetzt, unter Bildung des entsprechenden 3-Mercapto-4-C₁₋₆alkyl-1,2,4-triazolderivativs, das gegebenenfalls unter Bildung des entsprechenden 3-Methylmercaptoderivats methyliert wird, und anschließend die 3-Mercapto- bzw. 3-Methylmercaptogruppe entfernt, unter Bildung einer Verbindung der Formel (I), in welcher R⁴ für eine Gruppe der Formel (c-3) steht, in welcher R¹⁸ für eine C₁₋₆₋Alkylgruppe steht; oder mit einem 3-Brompyridylreagens umsetzt, unter Bildung einer Verbindung der Formel (I), in welcher R⁴ für eine Gruppe der Formel (c-4) steht; oder
e) eine Verbindung der Formel (VI): 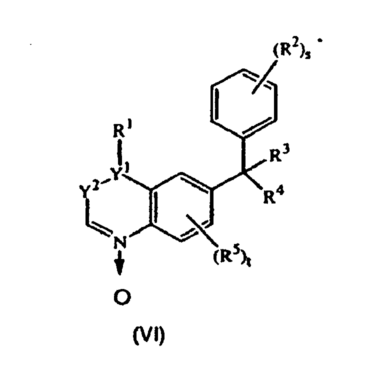 mit einem Reagens umsetzt, das dazu dient, diese Verbindung (VI) in eine Verbindung der Formel (I), in welcher R⁶ für Wasserstoff und R⁷ für Sauerstoff steht, umzuwandeln;
und gegebenenfalls in beliebiger Reihenfolge eine oder mehrere der folgenden Umwandlungen durchführt:
(i) Umwandlung einer Verbindung der Formel (I) in eine andere Verbindung der Formel (I) ;
(ii) Umwandlung einer Verbindung der Formel (I) in ein pharmazeutisch unbedenkliches Salz oder N-Oxid davon;
(iii) Umwandlung eines pharmazeutisch unbedenklichen Salzes oder N-Oxids einer Verbindung der Formel (I) in die Stammverbindung der Formel (I);
(iv) Herstellung einer stereochemisch isomeren Form einer Verbindung der Formel (I) oder eines pharmazeutisch unbedenklichen Salzes oder N-Oxids davon.

7. Verbindungen nach einem der Ansprüche 1 bis 5 zur Verwendung als Medizin.

8. Verbindungen nach Anspruch 7 zur Verwendung bei der Inhibierung von Tumorwachstum.

9. Verwendung einer Verbindung nach Anspruch 1 bei der Herstellung eines Medikaments zur Inhibierung von Tumorwachstum durch Verabreichen einer wirksamen Menge des Medikaments an einen Patienten, der einer solchen Behandlung bedarf.
